# EUROPÄISCHE PATENTANMELDUNG

(11) **EP 3 617 276 A1**
(43) Veröffentlichungstag der Anmeldung: **04.03.2020**
(21) Anmeldenummer: 19199245.2
(22) Anmeldetag: 25.11.2008
(51) Int. Cl.: C09C 1/00, A61K 8/25, A61Q 1/02, C08K 9/02, C09D 5/36, A61K 8/02, A61K 8/19, A61Q 1/04, A61Q 1/06, A61Q 1/10, A61Q 1/12, A61Q 3/02, A61Q 13/00, A61Q 17/04, A61Q 19/00, A61Q 19/10, A61Q 1/08

(54) **EFFEKTPIGMENTE BASIEREND AUF KÜNSTLICH HERGESTELLTEN SUBSTRATEN MIT ENGER GRÖSSENVERTEILUNG**

(30) Priorität: 20.02.2008 EP 08003114
(62) Teilanmeldung aus: 11171703.9
(71) Anmelder: ECKART GmbH, 91235 Hartenstein (DE)
(72) Erfinder: KAUPP, Günter, 91235 Hartenstein (DE); SCHMIDT, Ulrich, 91235 Hartenstein (DE); Schumacher, Dirk, 91235 Hartenstein (DE); SCHNEIDER, Ralf, 91235 Hartenstein (DE)
(74) Vertreter: Altana IP Department

(57) **Zusammenfassung**

Die Erfindung betrifft Effektpigmente, umfassend künstliche plättchenförmige Substrate, die mindestens eine optisch wirksame Beschichtung aufweisen, wobei die Effektpigmente eine Volumen-gemittelte Größensummendurchgangsverteilungskurve mit den Kennzahlen D₁₀, D₅₀ und D₉₀ aufweisen, wobei diese Größensummendurchgangsverteilungskurve einen Span ΔD von 0,7 -1,4 hat und der Span ΔD gemäß Formel (I) berechnet ist: $ΔD = \left({D}_{90}-{D}_{10}\right) / {D}_{50} ,$ und wobei die mittlere Dicke der künstlichen plättchenförmigen Substrate 500 nm bis 2000 nm und die Standardabweichung der Dicke der künstlichen Substrate 15 % bis 70 % beträgt.

## Beschreibung

Die vorliegende Erfindung betrifft neue, auf künstlichen Substraten basierende Effektpigmente mit verbesserten optischen Eigenschaften.

Außerdem betrifft die vorliegende Erfindung ein neues Verfahren zur Herstellung dieser Effektpigmente sowie deren Verwendung in Kosmetika, Farben, Druckfarben, Lacken, Pulverlacken und Kunststoffen.

Die optische Wirkung von Effektpigmenten beruht auf der gerichteten Reflexion von Licht an überwiegend flächig ausgebildeten, zueinander im Wesentlichen parallel ausgerichteten lichtbrechenden Pigmentteilchen. Diese Pigmentteilchen weisen in der Regel ein im Wesentlichen transparentes Substrat und eine oder mehrere Beschichtungen auf dem Substrat auf. Je nach Zusammensetzung der Beschichtung(en) der Pigmentteilchen erzeugen Interferenz-, Reflexions- und Absorptionsphänomene Farb- und Helligkeitseindrücke. Unregelmäßigkeiten in der zu beschichtenden Substratoberfläche oder farbige Verunreinigungen des Substrats können zu unerwünschten Streulichteffekten oder Farbunreinheiten im Endprodukt führen. Insbesondere bei Verwendung natürlicher Substratmaterialien, wie natürlichem Glimmer, treten diese unerwünschten Streulichteffekte und/oder farblichen Verunreinigungen auf. Künstliche Substrate bieten unter Umgehung genannter Nachteile neue Möglichkeiten für qualitativ hochwertige Pigmente mit neuen Farb- und Glanzeffekten.

In dem US Patent 3,331,699 A werden Perlglanzpigmente basierend auf Glasplättchen mit Interferenzfarben und intensivem Glitzereffekt beschrieben. Die Glasplättchen sind mit einer durchscheinenden hochbrechenden Metalloxidschicht beschichtet. Die Farbe der Pigmente ist abhängig vom gewählten Metalloxid und der Dicke der Metalloxidschicht.

Metallbeschichtete Glasplättchen, die gegen korrosive Einflüsse mit einer zusätzlichen Schutzschicht aus Metalloxiden wie SiO₂, Al₂O₃ oder TiO₂ belegt sind, werden in dem US-Patent 5,436,077 A beschrieben. In ihrem Aussehen sind diese beschichteten Glasplättchen vergleichbar mit Metallfolien.

Mit Titan- und/ oder Eisenoxiden beschichtete Glasplättchen sind aus dem US-Patent 6,045,914 A bekannt. Die durchschnittliche Teilchengröße der Glasplättchen liegt in einem Bereich von ungefähr 1 bis 250 µm, die Dicke bei ca. 0,1 bis 10 µm.

In der WO 2004/055119 A1 werden Interferenzpigmente auf der Basis von beschichteten plättchenförmigen Substraten beschrieben. Die Substrate sind hierbei mit einer ersten Schicht aus SiO₂ belegt, auf die anschließend eine hochbrechende Schicht, bestehend aus beispielsweise TiO₂, ZrO₂, SnO₂, Cr₂O₃, Fe₂O₃ oder Fe₃O₄ bzw. ein Interferenzsystem aus alternierenden hoch- und niedrigbrechenden Schichten aufgebracht ist. Optional können die Pigmente noch eine äußere Schutzschicht aufweisen.

Thermisch und mechanisch stabile metalloxidbeschichtete Effektpigmente basierend auf dünnen Glasplättchen mit einer Dicke ≤ 1,0 µm sind aus der WO 2002/090448 A2 bekannt.

Die optischen Eigenschaften von Effektpigmenten können nach der WO 2006/110359 A2 durch eine geeignete Partikelgrößenverteilung beeinflusst werden. Die hier beschriebenen klassierten metalloxidbeschichteten Glasplättchen weisen einen D₁₀ von wenigstens 9,5 µm, vorzugsweise von 9,5 µm, auf. Nachteilig ist, dass die Pigmente einen Größenbereich mit einem D₉₀-Wert von maximal 85 µm, vorzugsweise von etwa 45 µm, aufzuweisen haben. Mithin können gemäß der Lehre der WO 2006/110359 A2 nur kleinteilige Effektpigmente mit verbessertem Glitzereffekt bereitgestellt werden.

Anwendungstechnische Nachteile, wie beispielsweise die Verstopfung von Filtern, können gemäß der Lehre der WO 2007/114442 A1 durch eine geeignete Partikelgrößenverteilung vermieden werden, wobei der D₁₀ zwischen 4,7 und 25 µm und das Verhältnis von D₉₀ zu D₁₀ zwischen 2 und 3 liegt.

Bei den bislang im Stand der Technik bekannten Effektpigmenten ist nachteilig, dass diese nur eine geringe Farbreinheit aufweisen. So werden von einem Betrachter einer Mehrzahl an Effektpigmenten einer Charge unter dem Lichtmikroskop Effektpigmente mit verschiedenen, d.h. voneinander unterschiedlichen Farben, wahrgenommen.

Der vorliegenden Erfindung liegt die Aufgabe zugrunde, neue Effektpigmente mit verbesserten optischen Eigenschaften, insbesondere mit einer gegenüber dem Stand der Technik erhöhten Farbreinheit bei einem konstanten Lichteinfalls- und Beobachtungswinkel, bereitzustellen. Weiterhin ist es Aufgabe der Erfindung, ein Verfahren zur Herstellung dieser Effektpigmente zur Verfügung zu stellen.

Die Aufgabe wurde gelöst durch Bereitstellung von Effektpigmenten, umfassend künstliche plättchenförmige Substrate, die mindestens eine optisch wirksame Beschichtung aufweisen, wobei die Effektpigmente eine Volumen-gemittelte Größensummendurchgangsverteilungskurve mit den Kennzahlen D₁₀, D₅₀ und D₉₀ aufweisen, wobei diese Größensummendurchgangsverteilungskurve einen Span ΔD von 0,7 - 1,4 hat und der Span Δ D gemäß Formel (I) berechnet ist: $ΔD = \left({D}_{90}-{D}_{10}\right) / {D}_{50} ,$ wobei die mittlere Dicke der künstlichen plättchenförmigen Substrate 500 nm bis 2.000 nm beträgt.

Bevorzugte Weiterbildungen sind in den abhängigen Ansprüchen 2 bis 10 angegeben.

Die Aufgabe wurde weiterhin gelöst durch Bereitstellung eines Verfahrens zur Herstellung der erfindungsgemäßen Effektpigmente, welches folgende Schritte umfasst:
a) Größenklassieren der künstlichen Substrate,
b) Beschichten der künstlichen Substrate.

Vorzugsweise erfolgt das Beschichten der Substrate in Schritt b) nach dem Größenklassieren in Schritt a).

Des Weiteren ist Gegenstand der Erfindung die Verwendung der erfindungsgemäßen Effektpigmente in Kosmetika, Kunststoffen und Beschichtungszusammensetzungen, wie Farben, Druckfarben, Lacken, Pulverlacken und Elektrotauchlacken. Gegenstand der Erfindung sind mithin Zubereitungen, die die erfindungsgemäßen Effektpigmente enthalten.

Unter Effektpigmenten mit verbesserten optischen Eigenschaften werden im Sinne dieser Erfindung Effektpigmente verstanden, die sich insbesondere durch ihre hohe Farbreinheit bei einem konstanten Lichteinfalls- und Beobachtungswinkel auszeichnen. Die erfindungsgemäßen Effektpigmente weisen mithin in ihrer jeweiligen Gesamtheit eine einheitliche Farbe bzw. einen einheitlichen Farbton auf. Somit kommt es für einen Betrachter zu keinen merklichen farblichen Unterschieden von einem erfindungsgemäßen Effektpigment zu einem nächsten erfindungsgemäßen Effektpigment in einer Gesamtheit.

Überraschenderweise wurde gefunden, dass Substrate mit engem Span in der Teilchengrößenverteilung, die mit wenigstens einer optisch wirksamen Beschichtung beschichtet sind, eine deutlich höhere Farbreinheit gegenüber Effektpigmenten mit einem breiteren Span, wie dies im Stand der Technik der Fall ist, aufweisen.

Zur Charakterisierung der Teilchengrößenverteilung wird erfindungsgemäß der Span ΔD, definiert als ΔD = (D₉₀ - D₁₀)/D₅₀, verwendet. Je kleiner der Span ist, desto enger ist die Teilchengrößenverteilung.

Der D₁₀-Wert gibt den Wert der Längsausdehnung der Effektpigmente an, der mittels Lasergranulometrie als Kugeläquivalent ermittelt wird, den 10% der Pigmentteilchen der Gesamtheit aller Teilchen unterschreiten oder maximal aufweisen. Der D₅₀-Wert bzw. der D₉₀-Wert geben entsprechend die maximalen Längsausdehnungen der Effektpigmente an, die mittels Lasergranulometrie als Kugeläquivalente ermittelt werden, welche 50 % bzw. 90 % der Teilchen der Gesamtheit aller Teilchen unterschreiten oder maximal aufweisen.

Die erfindungsgemäßen Effektpigmente besitzen einen Span ΔD in einem Bereich von 0,7 bis 1,4, vorzugsweise von 0,7 bis 1,3, weiterhin bevorzugt von 0,8 bis 1,2, besonders bevorzugt von 0,8 bis 1,1.

Oberhalb eines Spans ΔD von 1,4 werden keine farbreinen Effektpigmente erhalten. Effektpigmente unterhalb eines Spans der Größenverteilung von 0,7 können im Rahmen der üblichen Methoden nur sehr aufwändig und damit nicht mehr wirtschaftlich hergestellt werden.

Die erfindungsgemäßen Effektpigmente können eine beliebige mittlere Teilchengröße (D₅₀) aufweisen. Die D₅₀-Werte der erfindungsgemäßen Effektpigmente umfassen einen Bereich von 3 bis 350 µm. Bevorzugt weisen die erfindungsgemäßen Effektpigmente einen D₅₀-Wert in einem Bereich von 3 - 15 µm oder 10 - 35 µm oder 25 - 45 µm oder 30 - 65 µm oder 40 - 140 µm oder 135 - 250 µm auf.

Die D₁₀-Werte der erfindungsgemäßen Effektpigmente umfassen vorzugsweise einen Bereich von 1 bis 120 µm. Bevorzugt weisen die erfindungsgemäßen Effektpigmente die in Tabelle 2 angegebenen Kombinationen von D₉₀-, D₅₀- und D₁₀-Werten auf. Hierbei werden die D₁₀, D₅₀ und D₉₀-Werte der Tabelle 2 nur in der Art kombiniert, daß sich ein Span ΔD von 0,7-1,4 ergibt. Kombinationen von D₁₀-, D₅₀- und D₉₀-Werten, die zu einem Span führen, der nicht im Bereich ΔD von 0,7 - 1,4 liegt, sind keine erfindungsgemäßen Ausführungsformen.

**Tabelle 2: Bevorzugte Kombination von Wertbereichen der D₉₀-, D₅₀- und D₁₀-Werte**

| D₉₀ (µm) | D₅₀ (µm) | D₁₀ (µm) |
|---|---|---|
| 8 - 25 | 3 - 15 | 1 - 5 |
| 20 - 45 | 10 - 35 | 5 - 25 |
| 40 - 70 | 25 - 45 | 10 - 30 |
| 70-110 | 30 - 65 | 20 - 45 |
| 120 - 180 | 40 - 140 | 25 - 65 |
| 400 - 490 | 135 - 250 | 75 - 110 |

Es hat sich überraschend gezeigt, dass die in Tabelle 2 angegebenen Kombinationen an Wertebereichen der D₉₀-, D₅₀- und D₁₀-Werte äußerst farbreine Effektpigmente ergeben.

Dabei hat sich überraschenderweise gezeigt, dass die absolute Größe der Effektpigmente nicht entscheidend ist, sondern das Verhältnis der Größen zueinander. Entscheidend ist, dass der Span ΔD = (D₉₀ - D₁₀)/ D₅₀ in einem engen Bereich von 0,7 bis 1,4 liegt. Dabei können beispielsweise die D₅₀-Werte der Effektpigmente bei 15, 20, 25, 30 µm oder auch bei 50, 80, 100, 150, 200, 250, 300 oder 350 µm liegen. Es hat sich gezeigt, dass überraschenderweise auch bei einer Mehrzahl von größeren Effektpigmenten farbreine Effektpigmente erhalten werden, wenn der Span ΔD in einem Bereich von 0,7 bis 1,4 liegt.

Die mittlere Dicke der künstlichen plättchenförmigen Substrate beträgt 500 nm bis 2.000 nm und bevorzugt 750 bis 1.500 nm.

Das Aspektverhältnis der erfindungsgemäßen Effektpigmente, berechnet aus dem Quotient von Teilchendurchmesser zu Dicke, liegt vorzugsweise bei 2- 980, bevorzugt bei 5 - 950 und besonders bevorzugt bei 10 - 920.

Beschichtet man Substrate unterhalb einer mittleren Dicke von 500 nm mit hochbrechenden Metalloxiden, so hat das Substrat einen deutlichen optischen Einfluss auf die Interferenzfarbe des Gesamtsystems. Daher erhält man Effektpigmente, die nicht mehr die gewünschte hohe Farbreinheit aufweisen. Außerdem nimmt die mechanische Stabilität dieser Effektpigmente beispielsweise gegenüber Scherkräften deutlich ab. Zudem dauern die Beschichtungszeiten dieser dünnen Substrate mit beispielsweise hochbrechenden Metalloxiden oder semitransparenten Metallen aufgrund der hohen spezifischen Oberflächen dieser Pigmente sehr lange, was hohe Herstellkosten verursacht.

Oberhalb einer mittleren Substratschichtdicke von 2.000 nm werden die Effektpigmente insgesamt zu dick. Damit einher geht eine schlechtere Deckfähigkeit sowie eine geringere planparallele Orientierung im Anwendungsmedium. Aus der schlechteren Orientierung wiederum resultiert ein verminderter Glanz.

Bei einer bevorzugten Ausführungsform beträgt die Standardabweichung der Dicke der künstlichen Substrate 15 % bis 100 % und besonders bevorzugt 20 % bis 70 %.

Die mittlere Dicke wird anhand eines gehärteten Lackfilmes, in dem die Effektpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die Dicke von 100 Effektpigmenten bestimmt und statistisch gemittelt wird.

Unterhalb einer Standardabweichung von 15 % werden farbflopende Effektpigmente erhalten. Oberhalb einer Standardabweichung von 100 % sind derart viele dickere Pigmente in dem gesamten Pigmentensemble enthalten, dass es dann zu schlechterer Orientierung und damit zu Glanzverlusten kommt.

Es hat sich überraschend gezeigt, dass Effektpigmente, deren künstliche Substrate eine mittlere Dicke von 500 bis 2000 nm und zugleich einen Span ΔD von 0,7 bis 1,4 aufweisen, die gewünschte außerordentliche Farbreinheit bei einem konstanten Lichteinfalls- und Beobachtungswinkel aufweisen.

Bei den Effektpigmenten handelt es sich vorzugsweise um Perlglanzpigmente oder um optisch variable Pigmente.

Die erfindungsgemäßen Effektpigmente können hierbei auch in Mischung mit weiteren Effektpigmenten vorliegen.

Effektpigmente auf der Basis von natürlichem Glimmer sind seit langem bekannt. Allerdings ist bei der Verwendung natürlichen Glimmers als Substrat zu beachten, dass dessen Oberfläche nicht ideal glatt ist, sondern Unregelmäßigkeiten, wie z.B. Stufen aufweist. Diese Unregelmäßigkeiten limitieren die Qualität der resultierenden Effektpigmente, da sie oft zu unterschiedlichen Farben innerhalb desselben Pigmentplättchens führen. Hierdurch wird insbesondere die Farbreinheit erheblich vermindert. Des weiteren kann durch im natürlichen Glimmer enthaltenen Verunreinigungen an Fremdionen der Farbeindruck der Effektpigmente verändert sein, so dass eine Mehrzahl von Effektpigmenten, die aus einer Produktionscharge stammen, voneinander verschiedene Farben oder Farbtöne aufweisen, mithin die Pigmentmischung eine geringe Farbreinheit aufweist.

Umgangen werden können diese Nachteile natürlichen Glimmers durch die Verwendung künstlicher Substrate. Unter künstlichen Substraten werden Substrate verstanden, die als solche in der Natur nicht vorkommen, sondern synthetisiert werden müssen. Geeignete Basissubstrate für die erfindungsgemäßen Effektpigmente sind beispielsweise synthetische, nichtmetallische, plättchenförmige Substrate. Die Substrate sind vorzugsweise im Wesentlichen transparent, bevorzugt transparent.

Gemäß einer bevorzugten Variante der Erfindung werden als Substrate Glasplättchen, Plättchen aus synthetischem Glimmer, SiO₂-Plättchen, Polymerplättchen, plättchenförmiges Bismuthoxychlorid und/oder plättchenförmige Aluminiumoxide oder Gemische davon verwendet. Bevorzugt werden für die erfindungsgemäßen Effektpigmente Substrate bestehend aus Glasplättchen oder synthetischem Glimmer eingesetzt.

Die erfindungsgemäßen Effektpigmente können Substrate sein, die mit einer oder mehreren optisch wirksamen Schichten oder Beschichtungen versehen sind. Unter einer optisch wirksamen Schicht oder Beschichtung wird eine Schicht bzw. eine Beschichtung verstanden, an der einfallendes Licht durch physikalische Effekte wie Reflexion, Interferenz, Absorption, Lichtbrechung, etc. wahrnehmbare Farbeffekte erzeugt.

Als optische wirksame Schichten oder Beschichtungen werden vorzugsweise Schichten aufgebracht, die Metalloxide, Metalloxidhydrate, Metallsuboxide, Metalle, Metallfluoride, Metallnitride, Metalloxynitride oder Mischungen davon umfassen. Gemäß einer bevorzugten Variante bestehen die optisch wirksamen Schichten oder Beschichtungen aus den vorstehend genannten Materialien.

Die Begriffe Schichten oder Beschichtungen werden im Sinne dieser Erfindung austauschbar verwendet, sofern nicht anders angegeben.

Gemäß einer bevorzugten Variante der Erfindung kann eine Beschichtung oder können mehrere Beschichtungen einen Brechungsindex n ≥ 1,9 aufweisen, mithin hochbrechend sein.

Wenn die erfindungsgemäßen Effektpigmente mehrere Schichten aufweisen, sind diese in Bezug auf den Brechungsindex vorzugsweise alternierend angeordnet, so das auf eine hoch brechende Schicht mit einem Brechungsindex von n ≥ 1,9 vorzugsweise eine niedrigbrechende Schicht mit einem Brechungsindex n < 1,9 folgt bzw. umgekehrt.

Die Beschichtung kann dabei entweder das gesamte Substrat umhüllen oder nur partiell auf dem Substrat vorliegen. Die Metalloxid-, Metalloxidhydrat-, Metallsuboxid-, Metall-, Metallfluorid-, Metallnitrid-, Metalloxynitridschichten oder Schichten mit Mischungen von den vorgenannten Verbindungen können niedrigbrechend (Brechungsindex < 1,9) oder hochbrechend (Brechungsindex n ≥ 1,9) sein.

Bei einer bevorzugten Ausführungsform umfasst oder ist das Substrat ein niedrig brechendes plättchenförmiges Substrat und die Beschichtung weist mindestens eine hochbrechende Schicht (n ≥ 1,9) auf. Vorzugsweise werden als Substratplättchen Glasplättchen, synthetische Glimmerplättchen und/oder SiO₂-Plättchen verwendet.

Als Metalloxide und/oder Metalloxidhydrate werden vorzugsweise Titanoxid, Titandioxid, Titanoxidhydrat, Aluminiumoxid, Aluminiumoxidhydrat, Siliziumoxid, Siliziumdioxid, Eisenoxid, Eisenhydroxid, Zinnoxid, Chromoxid, Antimonoxid, Ceroxid oder deren Mischoxide verwendet.

Bei einer weiteren erfindungsgemäßen Ausgestaltung der Erfindung sind die Substrate mit Metallschichten beschichtet. Dabei werden bevorzugt semitransparente Metallschichten aufgebracht, um Effektpigmente mit Interferenzfarben nach dem Fabry-Perot-Effekt bereitzustellen.

Geeignete Metalle zur Erzeugung der semitransparenten Metallschichten sind beispielsweise Aluminium, Chrom, Nickel, Silber, Gold, Titan, Kupfer oder deren Legierungen.

Die Schichtdicke der semitransparenten Metallschichten liegt bevorzugt in einem Bereich von 4 bis 40 nm und besonders bevorzugt 5 bis 30 nm.

Als Metallfluorid wird gemäß einer Variante Magnesiumfluorid als Beschichtung aufgebracht. Als Metallnitride oder Metalloxynitride können beispielsweise die der Metalle Titan, Zirkonium und/oder Tantal eingesetzt werden.

Bevorzugt wird das Substrat mit Metalloxid- und/oder Metalloxidhydratschichten beschichtet. Beispiele niedrigbrechender Schichten umfassen Aluminiumoxid, Aluminiumoxidhydrat, Siliziumoxid, Siliziumoxidhydrat und/oder Magnesiumfluorid.

Als hochbrechende Schichten werden beispielsweise Titandioxid, Titansuboxide, Eisenoxid, Eisenhydroxid, Zinnoxid, Zinkoxid, Zirkoniumoxid, Ceroxid, Cobaltoxid, Chromoxid, Antimonoxid oder deren Mischoxide eingesetzt.

Unterschiedliche Farben bei den erfindungsgemäßen Effektpigmenten sind hierbei durch Wahl des Schichtmaterials und der Schichtdicke einstellbar, wie Tabelle 1 beispielhaft zu entnehmen ist.

| | Belegung/ Schichtdicke | Farbe |
|---|---|---|
| Silberweiße Perlglanzpigmente Interferenzpigmente | TiO₂: 40 - 60 nm | silber |
| | TiO₂: 60 - 80 nm | gelb |
| | TiO₂: 80 - 100 nm | rot |
| | TiO₂: 100 - 140 nm | blau |
| | TiO₂: 120 - 160 nm | grün |
| | TiO₂: 280 - 320 nm | Grün (III. Ordnung) |
| Farbglanzpigmente | Fe₂O₃: 35 - 45 nm | bronze |
| | Fe₂O₃: 45 - 55 nm | kupfer |
| | Fe₂O₃: 55 - 65 nm | rot |
| | Fe₂O₃: 65 - 75 nm | rotviolett |
| | Fe₂O₃: 75 - 85 nm | rotgrün |
| | Fe₃O₄ | schwarz |
| Kombinationspigmente | TiO₂/ Fe₂O₃ | Goldtöne |
| | TiO₂/ Cr₂O₃ | grün |
| | TiO₂/ Berliner Blau | tiefblau |

Weisen die erfindungsgemäßen Effektpigmente eine Beschichtung mit Titandioxid auf, kann das Titandioxid in der Rutil- oder Anataskristallmodifikation vorliegen. Die qualitativ besten und stabilsten Perlglanzpigmente werden erhalten, wenn die Titandioxidschicht in der Rutilform vorliegt. Die Rutilform kann erhalten werden, indem beispielsweise vor Aufbringung der Titandioxidschicht eine Schicht aus SnO₂ auf das Substrat oder das Pigment aufgebracht wird. Auf einer Schicht aus SnO₂ kristallisiert TiO₂ bei Aufbringung in der Rutilmodifikation.

Die Dicke der Metalloxid-, Metalloxidhydrat-, Metallsuboxid-, Metall-, Metallfluorid- oder Metallnitridschichten oder von Schichten mit einer Mischung von den vorgenannten Verbindungen liegt üblicherweise in einem Bereich von 30 - 350 nm, weiter bevorzugt von 50 - 300 nm.

Die Metalloxid-, Metalloxidhydrat-, Metallsuboxid-, Metall-, Metallfluorid- und/oder Metallnitridschichten können Farbmittel wie Berliner Blau, Ultramarin, Karminrot, Azopigmenten, Phthalocyaninen oder FD & C Farbstoffen/-lacken enthalten oder aufweisen. Hierbei können die Farbmittel in der Beschichtung enthalten sein und/oder auf dieser Beschichtung aufgebracht und gegebenenfalls durch Haftvermittler auf diesen fixiert sein.

Um die erfindungsgemäßen Effektpigmente besser vor Witterungseinflüssen zu schützen, können diese zusätzlich mit einer äußeren Schutzschicht belegt sein. Diese umfaßt oder besteht bevorzugt aus einer oder zwei Metalloxidschichten der Elemente Si, Al oder Ce. Besonders bevorzugt ist hierbei eine Reihenfolge, bei der zunächst eine Ceroxidschicht aufgebracht ist, der dann eine SiO₂-Schicht folgt, wie in der WO 2006/021386 A1 beschrieben, die hiermit unter Bezugnahme aufgenommen ist.

Die äußere Schutzschicht kann des Weiteren an der Oberfläche organisch-chemisch modifiziert sein. Beispielsweise können ein oder mehrere Silane auf dieser äußeren Schutzschicht aufgebracht sein. Bei den Silanen kann es sich um Alkylsilane mit verzweigtkettigen oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Degussa, Rheinfelden, Deutschland, hergestellt und unter dem Handelsnamen "Dynasylan®" vertrieben. Weitere Produkte können von der Fa. OSi Specialties (Silquest®-Silane) oder von der Fa. Wacker, beispielsweise Standard- und α-Silane aus der GENIOSIL®-Produktgruppe, bezogen werden.
Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), 3-Mercaptopropyltri(m)ethoxysilan (Dynasylan MTMO oder 3201; Silquest A-189), 3-Glycidoxypropyltrimethoxysilan (Dynasylan GLYMO, Silquest A-187), tris-(3-Trimethoxysilylpropyl)isocyanurat (Silquest Y-11597), gamma-Mercaptopropyltrimethoxysilan (Silquest A-189), Bis-(3-Triethoxysilylpropyl)polysulfid (Silquest A-1289), Bis-(3-Triethoxysilyl)disulfid (Silquest A-1589), beta-(3,4-Epoxycyclohexyl)ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), (Methacryloxymethyl)tri(m)ethoxysilan (GENIOSIL XL 33, XL 36), (Methacryloxymethyl)(m)ethyldimethoxysilan (GENIOSIL XL 32, XL 34), Isocyanatomethyl)trimethoxysilan (GENIOSIL XL 43), (Isocyanatomethyl)methyldimethoxysilan (GENIOSIL XL 42), (Isocyanatomethyl)trimethoxysilan (GENIOSIL XL 43) 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3-Methacryloxypropymethyldimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan.

Es ist aber auch möglich, andere organofunktionelle Silane auf den erfindungsgemäßen Effektpigmenten zu verwenden.

Weiterhin lassen sich, beispielsweise kommerziell von Degussa erhältliche, wäßrige Vorhydrolysate einsetzen. Hierzu gehören u.a. wässriges, alkoholfreies Aminosilanhydrolysat (Dynasylan Hydrosil 1151), wässriges, alkoholfreies amino/alkylfunktionelles Siloxancooligomer (Dynasylan Hydrosil 2627), wässriges, alkoholfreies diamino/alkylfunktionelles Siloxancooligomer (Dynasylan Hydrosil 2776), wässriges, alkoholfreies amino/vinylfunktionelles Siloxancooligomer (Dynasylan Hydrosil 2907), wässriges, alkoholfreies amino/alkylfunktionelles Siloxancooligomer (Dynasylan Hydrosil 2909), wässriges, alkoholfreies epoxyfunktionelles Siloxanoligomer (Dynasylan Hydrosil 2926) oder wässriges, alkoholfreies amino/methacrylatfunktionelles Siloxancooligomer (Dynasylan Hydrosil 2929), oligomeres Diaminosilansystem (Dynasylan 1146), vinyl-/alkylfunktionelles Siloxancooligomer (Dynasylan 6598), vinyl- und methoxygruppenhaltiges Vinylsilankonzentrat (oligomeres Siloxan) (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Effektpigmentes an verschiedenartige Bindemittel sehr gut geeignet.

Bevorzugt werden hierzu folgende Verbindungen genommen:
Aminopropyltrimethoxysilan (Dynasylan AMMO; Silquest A-1110), Aminopropyltriethoxysilan (Dynasylan AMEO) oder N-(2-Aminoethyl)-3-aminopropyltrimethoxysilan (Dynasylan DAMO, Silquest A-1120) oder N-(2-Aminoethyl)-3-aminopropyltriethoxysilan, Triamino-funktionelles Trimethoxysilan (Silquest A-1130), bis-(gamma-Trimethoxysilylpropyl)amin (Silquest A-1170), N-ethyl-gamma-aminoisobutyltrimethoxysilan (Silquest A-Link 15), N-Phenyl-gammaaminopropyltrimethoxysilan (Silquest Y-9669), 4-Amino-3,3-dimethylbutyltrimethoxysilan (Silquest Y-11637), N-Cyclohexylaminomethylmethyldiethoxysilan (GENIOSIL XL 924), (N-Cyclohexylaminomethyl)triethoxysilan (GENIOSIL XL 926), (N-Phenylaminomethyl)trimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel (A) auf:

R_{(4-z)}Si(X)_{z}, (A)

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

Ein derartiges Silan bewirkt eine starke Hydrophobierung der Pigmentoberfläche. Diese wiederum führt dazu, daß das derart beschichtete Perlglanzpigment in der Lackbeschichtung tendenziell nach oben aufschwimmt. Bei plättchenförmigen Effektpigmenten wird ein derartiges Verhalten als "leafing"-Verhalten bezeichnet.

Eine Silanmischung bestehend aus mindestens einem Silan, welches wenigstens eine funktionelle Gruppe besitzt, die eine Anbindung an das Bindemittel ermöglicht, und einem, in Wasser unlöslichen oder kaum löslichen Alkylsilan ohne Aminogruppe ermöglicht optimale anwendungstechnische Eigenschaften der Perlglanzpigmente.
Eine solche organisch-chemischen Oberflächenmodifizierung führt dazu, dass sich die Effektpigmente in einer Lack- oder Farbschicht ausgezeichnet orientieren, d.h. im Wesentlichen planparallel zu dem lackierten bzw. angestrichenen Untergrund, und zugleich chemisch mit dem Bindemittelsystem des Lackes bzw. der Farbe reagieren und mithin kovalent in der Lack- bzw. Farbschicht gebunden sind. Derartige Lack- bzw. Farbschichten weisen eine erhöhte mechanische und chemische Beständigkeit gegenüber Umwelteinflüssen, wie Wetter etc., auf.

Werden die erfindungsgemäßen Effektpigmente in einem Rakelabzug (z.B. im Nitrocelluloselack) unter einem Lichtmikroskop in der Durchsicht betrachtet, so erkennt man bei den erfindungsgemäßen Effektpigmenten, dass sie eine sehr einheitliche Farbe aufweisen. Die einzelnen Pigmente weisen weitgehend die gleiche Farbe, vorzugsweise die gleiche Farbe, auf.

Die Farbgebung von Effektpigmenten, wie Perlglanzpigmenten, erfolgt durch Interferenzphänomene. Einander komplementäre Interferenzfarben löschen sich dabei gegenseitig aus, so erzeugt beispielsweise eine Mischung von blauen und gelben Perlglanzpigmenten einen silbernen oder grauen Farbeindruck. Gut bekannt ist, dass Mischungen verschiedenfarbiger Perlglanzpigmente stets einen unreineren Farbton mit geringerem Glanz ergeben, da sich immer einige Farbanteile gegenseitig auslöschen bzw. schwächen.

Um Effektpigmente, vorzugsweise Perlglanzpigmente, mit hoher Farbreinheit zu erhalten, müssen mithin die Farbtöne der einzelnen Pigmente möglichst einheitlich sein. Die Farbtonreinheit kann sich durch folgendes Verfahren nach einer statistischen Auswertung quantitativ darstellen lassen.

Ausgangspunkt zur Bestimmung der Farbreinheit der erfindungsgemäßen Effektpigmente sind farbige digitale Mikroskopbilder von Rakelabzügen. Der Rakelabzug wird mit einem Lack, enthaltend 6 Gew.-%-Effektpigmente in einem farblosen Nitrocelluloselack, durchgeführt. Die Mengenangaben Gew.-% bezieht sich dabei auf das Gesamtgewicht des Lackes. Der Lack wird in einer Naßfilmdicke von 76 µm auf eine BYK-Gardner Schwarz-Weiß-Rakelkarte (byko-chart 2853) aufgebracht und nachfolgend getrocknet. Innerhalb dieser ausgetrockneten Rakelabzüge sind die plättchenförmigen Effektpigmente weitgehend planparallel orientiert. Aufgenommen werden die Mikroskopbilder bevorzugt mit dem Mikroskop Axioskop 2 und der Digitalkamera AxioCam der Fa. Zeiss, Deutschland. Von den erhaltenen Digitalbildern werden sodann mit Hilfe der Bildaufnahme- und Bildverarbeitungs-Software AxioVision 4.6 der Fa. Zeiss farbneutrale Digitalbilder mit 12 Bit Farbtiefe angefertigt. Bei der Betrachtung der Rakelabzüge durch das Mikroskop fällt das eingestrahlte Licht von oben senkrecht auf den Rakelabzug. In dem Rakelabzug wiederum sind die Effektpigmente weitgehend planparallel orientiert und mithin fällt das einfallende Licht senkrecht auf die Pigmentplättchen. Der Beobachtungswinkel ist in diesem Fall ebenfalls senkrecht zur Oberfläche der Pigmentplättchen.

Um eine verfälschungsfreie Statistik zu erhalten, wurden ca. 20 Einzelaufnahmen an zufällig ausgewählten Stellen auf dem schwarzen Untergrund der Rakelabzüge erstellt. Dabei wurden über 100 auswertbare Einzelpigmente abgebildet.

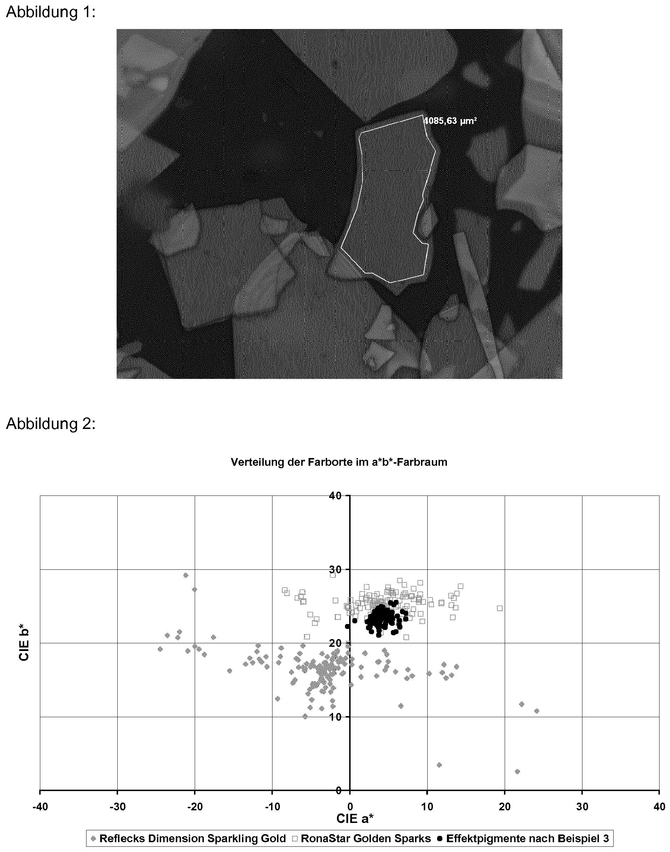

Da die Bildverarbeitungssoftware die Pigmente nicht automatisch erkennen kann, werden diese manuell markiert. Bei der Markierung der einzelnen Pigmentflächenanteile ist zu beachten, dass keine Pigmentrandbereiche bzw. Bereiche, in denen sich die Pigmente überlappen, genommen werden, da in diesem Fall der Farbeindruck der einzelnen Pigmentteilchen durch die Überlappung sofort verfälscht ist (s. auch Abbildung 1).

Bei den so markierten Flächen berechnet die Bildverarbeitungssoftware die jeweiligen Flächenmittelwerte der Rot-, Grün- und Blauanteile. Um eine aussagefähige Statistik zu werden die Farbanteile von mindestens 100 auswertbaren Pigmentteilchen aus den verschiedenen Mikroskopaufnahmen bestimmt.

In der Bildverarbeitungssoftware wird das sRGB-Farbmodell verwendet. Die Umrechnung in den XYZ-Farbraum erfolgt über die allgemein bekannte Transformationsmatrix (II) (IEC 61966-2-1:1999). $\left(\begin{matrix}X \\ Y \\ Z\end{matrix}\right) = \left(\begin{matrix}0 , 4124 & 0 , 3576 & 0 , 1805 \\ 0 , 2126 & 0 , 7152 & 0 , 0722 \\ 0 , 0193 & 0 , 1192 & 0 , 9505\end{matrix}\right) \left(\begin{matrix}R \\ G \\ B\end{matrix}\right)$

Die so erhaltenen X, Y und Z-Werte werden anschließend in die gebräuchlichen CIE (1976) Lab-Werte umgerechnet: $L * = 116 Y * - 16$ $a * = 500 \left(X*-Y*\right)$ $b * = 200 \left(Y*-Z*\right)$ $X * = \sqrt[3]{\frac{X}{{X}_{n}}}$

Die Berechnung für Y* und Z* erfolgt analog der Berechnung von X*. Es wurden die Normfarbwerte Xₙ = 94,81,Yₙ = 100 und Zₙ = 107,34 für die Lichtart D65 und den 10°-Normalbeobachter verwendet.

Man erhält so eine Verteilung von Meßpunkten im a*-b*-Farbraum (s. auch die Abbildung 2): Durch den Abstand der Meßpunkte vom Ursprung des a*,b*-Diagramms ist die Farbsättigung (Chroma) C* jedes einzelnen Meßwertes festgelegt.

Zur Quantifizierung der Streuung wird zunächst der gemeinsame Mittelpunkt (*a̅,b̅*) der Messwerte im a*-b*-Farbraum nach den Formeln (Vlla; Vllb) berechnet: $\overline{a} = {\sum }_{i} {a}_{i}$ $\overline{b} = {\sum }_{i} {b}_{i}$

Anschließend wird für jeden Meßpunkt (a,b) dessen Abstand zu dem Mittelpunkt (*a̅,b̅*) als Absolutwert Δ*C* * in Form der Wurzel des Quadrates der Differenz der Werte ermittelt (VIII): $Δ {C}^{*} = \sqrt{{\left(a-\overline{a}\right)}^{2} + {\left(b-\overline{b}\right)}^{2}}$

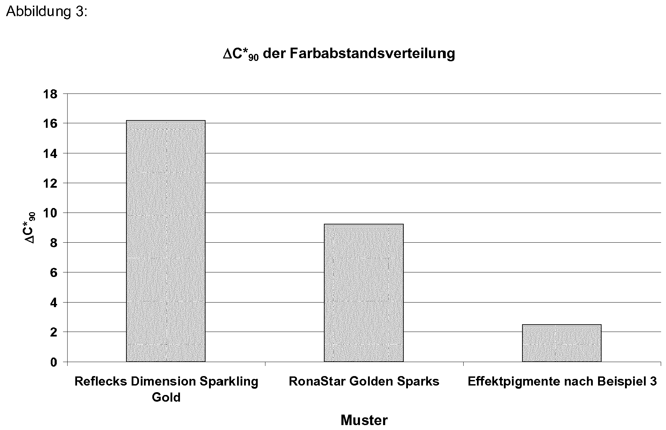

Man erhält eine Farbabstandsverteilung im a*-b*-Farbraum. Diese lässt sich ebenfalls als Summendurchgangsverteilungskurve darstellen. Das 90%-Quantil (ΔC*₉₀) dieser Farbabstandsverteilung ist ein geeignetes Maß, um die Größe der Farbstreuung zu charakterisieren (s. Abbildung 3). Dieser Wert markiert jenen Farbabstand ΔC* vom Mittelwert unterhalb dessen 90 % der gezählten Pigmentteilchen liegen.

Die erfindungsgemäßen Effektpigmente weisen vorzugsweise ein 90%-Quantil der Farbabstandsverteilung ΔC*₉₀ von 0,2 bis 8,0 und bevorzugt von 0,5 bis 6,0; weiter bevorzugt von 0,6 bis 5,0; besonders bevorzugt von 0,7 bis 4,0 und ganz besonders bevorzugt von 0,7 bis 3,0 auf.

Die Erfinder haben für diesen überraschenden Effekt noch keine wissenschaftlich aussagefähige Erklärung gefunden. Es wird jedoch vermutet, dass die in ihrer Größe weitgehend gleichen Pigmentsubstrate wesentlich gleichmäßiger mit den farbgebenden Schichten wie beispielsweise hochbrechende Metalloxide oder semitransparente Metalle beschichtet werden, als dies bei Substraten mit höherem Span ΔD der Fall ist. Dadurch bilden sich auf den Substraten Beschichtungen mit einer wesentlich homogeneren Schichtdicke aus. Da die Farbgebung durch Interferenzphänomene erzeugt und diese bei den verwendeten Substraten entscheidend von der Schichtdicke der hochbrechenden Beschichtung bestimmt wird, ergeben die gleichmäßigeren Schichtdicken einen reineren Farbton bzw. eine größere Farbreinheit bei den erfindungsgemäßen Effektpigmenten.

Die vorliegende Erfindung umfasst Perlglanzpigmente ohne Farbflop sowie auch Effektpigmente mit einem hohen Farbflop bei Multilayersystemen.

Entscheidend ist jedoch, dass bei konstantem Einfallswinkel und Beobachtungswinkel in einem Rakelabzug bei weitgehend planparalleler Orientierung der erfindungsgemäßen plättchenförmigen Effektpigmente stets ein weitgehend gleicher Farbton im Sinne der vorstehenden Beschreibung beobachtet wird. Dabei muß der Einfalls- bzw. Beobachtungswinkel keineswegs immer senkrecht zu den Pigmentplättchen sein, wenngleich dieser Fall auch bevorzugt ist.

Die erfindungsgemäßen Effektpigmente eignen sich insbesondere für die Anwendung in Kosmetika, wie z.B. Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen sowie Nagellackkompositionen.

Zur Erzielung spezieller Farbeffekte können in den Kosmetikapplikationen neben den erfindungsgemäßen Effektpigmenten weitere Farbmittel und/oder herkömmliche Effektpigmente oder Mischungen hiervon in variablen Mengenverhältnissen eingesetzt werden. Als herkömmliche Effektpigmente können beispielsweise handelsübliche Perlglanzpigmente auf der Basis von mit hochbrechenden Metalloxiden beschichtetem natürlichen Glimmer (wie z.B. die Produktgruppe Prestige® der Fa. Eckart), BiOCI-Plättchen, TiO₂-Plättchen, Perlglanzpigmente auf der Basis von mit hochbrechenden Metalloxiden beschichtetem synthetischen Glimmer oder basierend auf mit hochbrechenden Metalloxiden beschichteten Glasplättchen, Al₂O₃-, SiO₂- oder TiO₂-Plättchen verwendet werden. Außerdem können auch Metalleffektpigmente, wie z.B. die Produktgruppe Visionaire® der Fa. Eckart, zugegeben werden. Die Farbmittel können aus anorganischen oder organischen Pigmenten ausgewählt sein.

Bevorzugte Effektpigmente im Sinne der vorliegenden Erfindung besitzen einen Span ΔD von 0,7 - 1,4, vorzugsweise 0,7 - 1,3, weiter bevorzugt 0,8 - 1,2 und ganz besonders bevorzugt 0,8 - 1,1 sowie einen D₅₀-Wert von 3 - 350 µm. Ihre mittlere Dicke beträgt dabei 500 - 2000 nm, die Standardabweichung in der Dicke beträgt dabei 15 - 100 %.

Weiter bevorzugte Effektpigmente im Sinne der vorliegenden Erfindung besitzen einen Span Δ D von 0,8 - 1,2, einen D₅₀-Wert von jeweils vorzugsweise 3 - 15 µm, 10 - 35 µm, 25 - 45 µm, 30 - 65 µm, 40 - 140 µm oder 135 - 250 µm. Ihre mittlere Dicke beträgt dabei 500 - 2000 nm, und die Standardabweichung in der Dicke beträgt dabei 15 - 100%.

Weiter bevorzugte Effektpigmente im Sinne der vorliegenden Erfindung besitzen einen Span Δ D von 0,8 - 1,2 sowie eine mittlere Dicke von 500 - 2000 nm, besonders bevorzugt von 750 - 1.500 nm. Die Standardabweichung in der Dicke beträgt dabei 15 - 100 % und die Effektpigmente weisen dabei einen D₅₀-Wert von 3 - 350 µm auf.

Weiter bevorzugte Effektpigmente im Sinne der vorliegenden Erfindung besitzen einen Span Δ D von 0,8 - 1,2 sowie eine Standardabweichung in der Dicke von 20 - 70 %. Die mittlere Dicke der Effektpigmente beträgt dabei 500 - 2000 nm, bevorzugt 750 - 1500 nm. Sie weisen dabei einen D₅₀-Wert von 3 - 350 µm auf.

Ein Verfahren zur Herstellung der erfindungsgemäßen Effektpigmente umfasst die folgenden Schritte:
a) Größenklassieren der künstlichen Substrate
b) Beschichten der künstlichen Substrate.

Wenn die Ausgangssubstrate zu groß sind, kann optional ein Zerkleinerungsschritt vor dem Größenklassieren durchgeführt werden.

Die Größenklassierung kann vor oder nach der Beschichtung der Substrate erfolgen. Vorteilhafterweise wird jedoch zuerst das Substrat klassiert und anschließend beschichtet. Die Größenklassierung wird durchgeführt und gegebenenfalls wiederholt, bis die Perlglanzpigmente die erfindungsgemäße Teilchengrößenverteilung aufweisen.

Ein enger Span ΔD der Substrate kann durch geeignete Zerkleinerungs- und/oder Klassierungsprozesse der zu beschichtenden, künstlichen Substrate erreicht werden. Die zu beschichtenden künstlichen Substrate können beispielsweise durch Kugelmühle, Strahl- oder Rührwerkskugelmühle, Kollergang oder Dissolver zerkleinert werden. Der Span ΔD der Endfraktion kann durch geeignete Klassierung, wie beispielsweise eine mehrfache Naßsiebung, eingestellt werden. Weitere Klassierungsverfahren umfassen die Zentrifugation in Zyklonen oder eine Sedimentation aus einer Dispersion.

Die Zerkleinerungs- und Klassierprozesse können nacheinander erfolgen und gegebenenfalls miteinander kombiniert werden. So kann auf einen Zerkleinerungsvorgang ein Klassierungsvorgang folgen, dem sich ein weiterer Zerkleinerungsvorgang des Feinguts anschließt usw..

Die der Erfindung zugrundeliegende Aufgabe wird ferner durch Bereitstellung einer Beschichtungszusammensetzung, die Effektpigmente nach einem der Ansprüche 1 bis 10 enthält, gelöst. Gemäß einer bevorzugten Variante wird die Beschichtungszusammensetzung aus der Gruppe, die aus Kosmetikum, Farbe, Druckfarbe, Lack, Pulverlack und Elektrotauchlack besteht, ausgewählt.

Die der Erfindung zugrundeliegende Aufgabe wird ferner durch Bereitstellung eines Kunststoffs, der Effektpigmente nach einem der Ansprüche 1 bis 10 enthält, gelöst.

Im Folgenden wird die Erfindung durch einige Beispiele und Abbildungen näher erläutert, ohne auf diese beschränkt zu sein.

### Abbildungen

Abb. 1 zeigt einen manuell markierten Flächenanteil in einem Digitalbild eines erfindungsgemäßen Effektpigmentes in einem getrockneten Rakelauszug.

Abb. 2 zeigt die Verteilung der aus den Digitalbildern der manuell markierten Effektpigmente berechneten Farborte in einem a*b*-Farbraum.

Abb. 3 zeigt das 90 % Quantil ΔC*₉₀ der Farbabstandsverteilung

Die folgenden Beispiele sollen die Erfindung näher erläutern, ohne sie jedoch auf diese einzuschränken.

### Beispiel 1:

Eine Suspension von 200 g Glasplättchen (mittlere Dicke: 1 µm, Standardabweichg der Dicke: ca. 40%) in VE-Wasser (ca. 3 Gew.-%ig) (VE: vollentsalzt) wird über ein 100 µm Sieb klassiert und der Siebdurchgang wiederum über ein 63 µm Sieb gesiebt. Diese Siebprozedur wird mit auf dem 63 µm Sieb erhaltenen Siebrückstand zweimalig wiederholt und so wird eine Glasplättchenfraktion erhalten, die folgende Partikelgrößenverteilung aufweist (MALVERN Mastersizer MS 2000): D₁₀=50 µm, D₅₀=82 µm, D₉₀=132 µm, Span ΔD = 1,00.

### Beispiel 2:

200 g Glasplättchen aus Beispiel 1 werden in 2000 ml VE-Wasser suspendiert und unter turbulentem Rühren auf 80 °C erhitzt. Der pH-Wert der Suspension wird mit verdünnter HCl auf 1,9 eingestellt und sodann eine erste Schicht "SnO₂" auf den Glasplättchen gefällt. Diese Schicht wird durch Zugabe von einer Lösung, bestehend aus 3 g SnCl₄ x 5 H₂0 (in 10 ml konz. HCl plus 50 ml VE-Wasser), unter gleichzeitiger Zudosierung von einer 10% NaOH, um den pH-Wert konstant zu halten, über einen Zeitraum von 1 h gebildet. Um die Fällung zu vervollständigen wird die Suspension noch weitere 15 min gerührt. Danach wird der pH-Wert mit verdünnter HCl auf pH 1,6 heruntergesetzt und sodann eine Lösung von 200 ml TiCl₄ (400 gTiCl₄/l VE-Wasser) in die Suspension hinzudosiert. Der pH-Wert wird dabei durch Gegensteuern mit 10% NaOH konstant auf pH 1,6 gehalten. Danach wird noch 15 min nachgerührt, abfiltriert und der Filterkuchen mit VE-Wasser nachgewaschen. Der Filterkuchen wird dann bei 100 °C vorgetrocknet und bei 750 °C 30 min kalziniert. Es wird ein hochglänzendes Effektpigment erhalten, welches eine rote Interferenzfarbe zeigt. Eine mikroskopische Untersuchung der beschichteten Pigmente zeigt eine außergewöhnliche Farbkonstanz der Pigmente untereinander.

### Beispiel 3:

200 g Glasplättchen aus Beispiel 1 werden anlog Beispiel 2 mit TiO₂ beschichtet, allerdings werden nur 167 ml TiCl₄ zudosiert. Man erhält nach Kalzination ein hochglänzendes, Effektpigment mit einer goldenen Interferenzfarbe. Eine mikroskopische Untersuchung zeigt analog zu Beispiel 2 eine außergewöhnliche Farbkonstanz der Pigmente untereinander.

### Vergleichsbeispiel 4:

Kommerziell erhältliche goldene Perlglanzpigmente "Reflecks Dimension Sparkling Gold" der Fa. BASF Catalysts.

### Vergleichsbeispiel 5:

Kommerziell erhältliche goldene Perlglanzpigmente "RonaStar Golden Sparks" der Fa. Merck.

**Tab. 3: Kennwerte der Größenverteilung für einige Beispiele und Vergleichsbeispiele:**

| Pigment | D₁₀ | D₅₀ | D₉₀ | Span ΔD |
|---|---|---|---|---|
| Ronastar Golden Sparks (Vergleichsbeispiel 4) | 34,0 | 78,4 | 148,7 | 1,46 |
| Reflecks Dimension Sparkling Gold (Vergleichsbeispiel 5) | 35,6 | 84,3 | 175,9 | 1,66 |
| Beispiel 3 | 49,8 | 81,5 | 130,7 | 0,99 |

Im Folgenden wurden die erfindungsgemäßen Effektpigmente des Beispiels 3 mit engem Span und hoher Farbreinheit mit den kommerziell erhältlichen Perlglanzpigmenten der Vergleichsbeispiele in einer detaillierten Analyse verglichen. Alle Perlglanzpigmente weisen im Rakelabzug eine für den Beobachter visuell wahrnehmbare goldene Interferenzfarbe auf.

Ausgangspunkt zur Bestimmung der Farbreinheit der erfindungsgemäßen Effektpigmente waren farbige digitale Mikroskopbilder von getrockneten Rakelabzügen (hergestellt mit einem Lack, der 6 Gew.-%-Pigmente in Nitrocelluloselack enthält (Gew.-% Angabe bezieht sich auf das Gesamtgewicht des Lackes) und in einer Naßfilmdicke von 76 µm auf BYK-Gardner Schwarz-Weiß-Rakelkarte (byko-chart 2853) aufgerakelt und nachfolgend bei Raumtemperatur getrocknet wurden). Aufgenommen wurden diese mit dem Mikroskop Axioskop 2 und der Digitalkamera AxioCam der Fa. Zeiss. Diese Ausstattung erlaubte es, mit Hilfe der Bildaufnahme- und Bildverarbeitungs-Software AxioVision 4.6 farbneutrale Digitalbilder mit 12 Bit Farbtiefe anzufertigen.

Um eine verfälschungsfreie Statistik zu erhalten, wurden ca. 20 Einzelaufnahmen an zufällig ausgewählten Stellen der Rakelabzüge erstellt. Dabei wurden über 100 auswertbare Einzelpigmente abgebildet.

In Abb. 2 sind die Farborte der Einzelmessungen der drei Proben im a*,b*-Diagramm aufgetragen. Deutlich zu erkennen ist, dass die erfindungsgemäßen Effektpigmente im Unterschied zu den kommerziell erhältlichen Produkten die geringste Streuung im a*-b*-Farbraum aufweisen, also am farbreinsten sind. Die Farbwerte liegen dicht beisammen im rot-gelben Quadranten des a*-b*-Farbraums. Die größte Streuung, also die am stärksten uneinheitlich gefärbten Pigmente sind bei den Pigmenten des Vergleichsbeispiels 4 zu beobachten. Dies ist auch bereits mit bloßem Auge an den Rakelabzügen zu erkennen. Eine etwas engere Streuung, aber dennoch eine Verteilung über den grün- und rot-gelben Quadranten des a*-b*-Farbraums ist bei den Merck Pigmenten Ronastar (Fa. Merck, Darmstadt, Deutschland) vorhanden.

**Tabelle 4: ΔC*₉₀-Quantil**

| Pigment | ΔC*₉₀-Quantil |
|---|---|
| Ronastar Golden Sparks (Vergleichsbeispiel 4) | 9,2 |
| Reflecks Dimension Sparkling Gold (Vergleichsbeispiel 5) | 16,2 |
| Beispiel 3 | 2,5 |

Das 90%-Quantil der Farbabstandsverteilung ist für die erfindungsgemäßen Effektpigmente des erfindungsgemäßen Beispiels 3 am geringsten, während die kommerziell erhältlichen Vergleichsprodukte deutlich größere Werte aufweisen.

### Beispiel 6:

200 g Glasplättchen aus Beispiel 1 werden in 2000 ml VE-Wasser suspendiert und unter turbulentem Rühren auf 80 °C erhitzt. Der pH-Wert der Suspension wird mit verdünnter HCl auf pH 1,9 eingestellt und sodann eine erste Schicht "SnO₂" auf den Glasplättchen gefällt. Diese Schicht wird durch Zugabe von einer Lösung, bestehend aus 3 g SnCl₄ x 5 H₂0 (in 10 ml konz. HCl plus 50 ml VE-Wasser), unter gleichzeitiger Zudosierung von einer 10% NaOH, um den pH-Wert konstant zu halten, über den Zeitraum von 1 h gebildet. Um die Fällung zu vervollständigen wird die Suspension noch weitere 15 min gerührt. Danach wird der pH-Wert mit verdünnter HCl auf pH 1,6 heruntergesetzt und sodann eine Lösung von 185 ml TiCl₄ (400 gTiCl₄/l VE-Wasser) in die Suspension hinzudosiert. Der pH-Wert wird dabei durch Gegensteuern mit 10% NaOH konstant auf pH 1,6 gehalten. Danach wird der pH-Wert mit 5 % NaOH auf 7,5 angehoben und 15 min gerührt. Eine Wasserglaslösung (207 g Wasserglaslösung, 27% SiO₂, gemischt mit 207 g VE-Wasser) wird sodann langsam in die Suspension eingeleitet und der pH-Wert konstant bei pH 7,5 gehalten. Danach wird noch 20 min nachgerührt und der pH-Wert wieder auf 1,9 gesenkt. Dann scheidet man eine zweite Schicht "SnO₂" auf der SiO₂-Oberfläche ab. Diese Schicht wird durch Zugabe von einer Lösung, bestehend aus 3 g SnCl₄ x 5H₂0 (in 10 ml konz. HCl plus 50 ml VE-Wasser), unter gleichzeitiger Zudosierung von einer 10% NaOH, um den pH-Wert konstant zu halten, über den Zeitraum von 1 h gebildet. Um die Fällung zu vervollständigen wird die Suspension noch weitere 15 min gerührt. Danach wird der pH-Wert mit verdünnter HCl auf pH 1,6 heruntergesetzt und sodann eine Lösung von 189 ml TiCl₄ (400 gTiCl₄/l VE-Wasser) in die Suspension hinzudosiert. Der pH-Wert wird dabei durch Gegensteuern mit 10 % NaOH konstant auf pH 1,6 gehalten. Danach wird noch 15 min nachgerührt, abfiltriert und der Filterkuchen mit VE-Wasser nachgewaschen. Der Filterkuchen wird bei 100 °C vorgetrocknet und bei 750 °C 30 min kalziniert. Es wird ein extrem hochglänzendes Effektpigment erhalten, welches einen Farbflop aufweist, der von einer grünen Interferenzfarbe für steile Betrachtungswinkel in eine blaue Interferenzfarbe bei flachem Betrachtungswinkel wechselt.

Im Folgenden werden kosmetische Applikationen mit den erfindungsgemäßen Effektpigmenten vorgestellt:

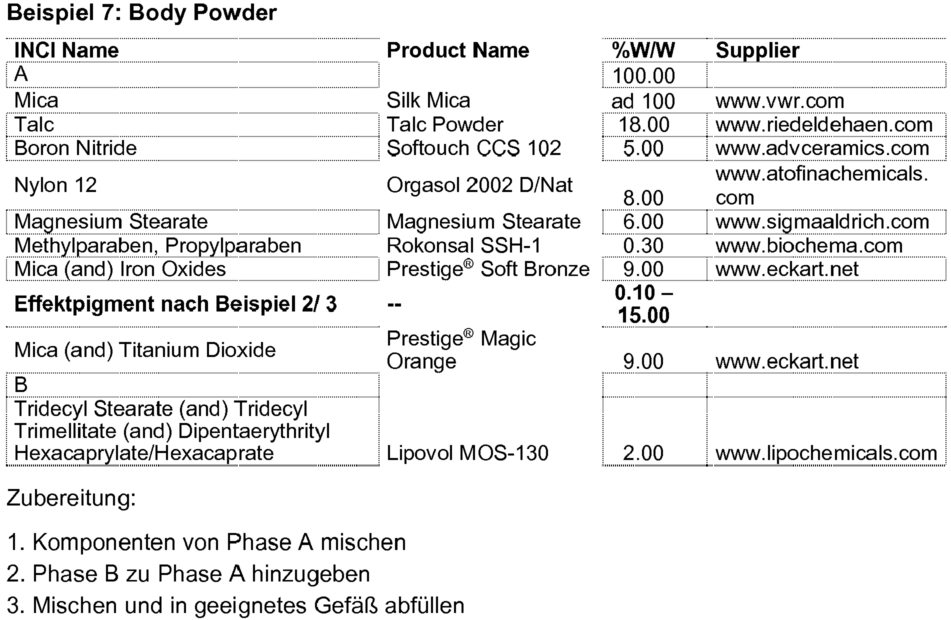

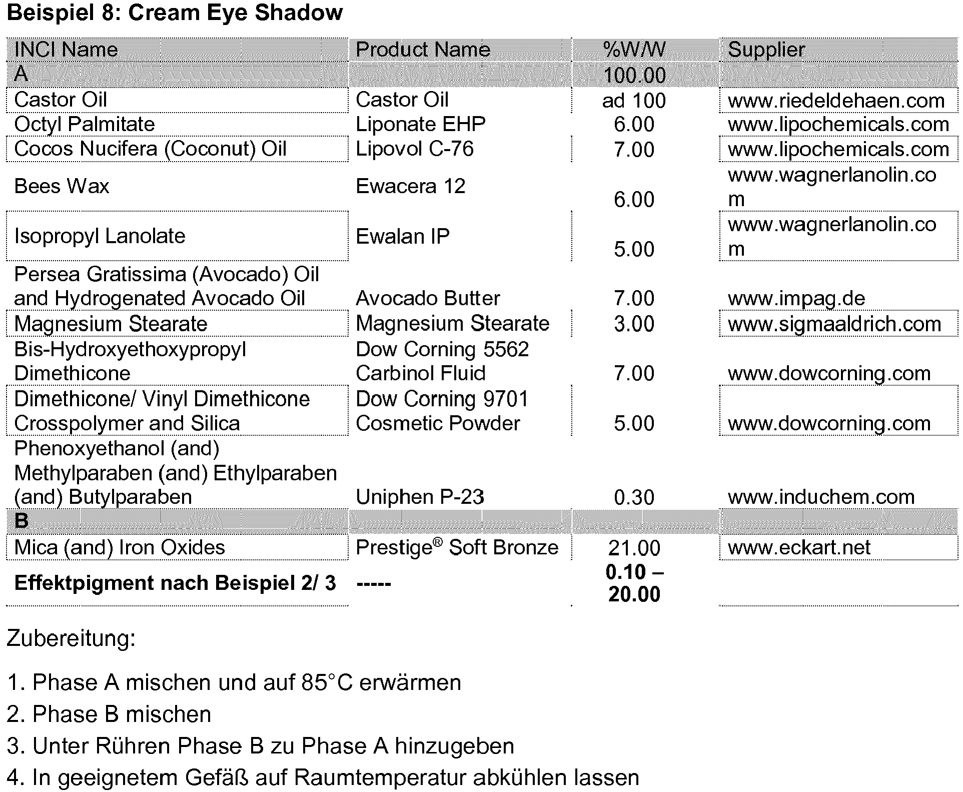

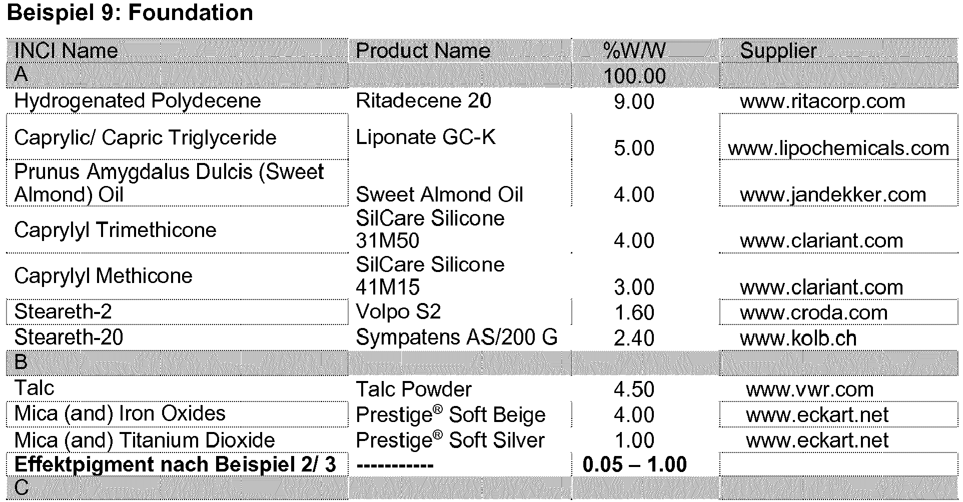

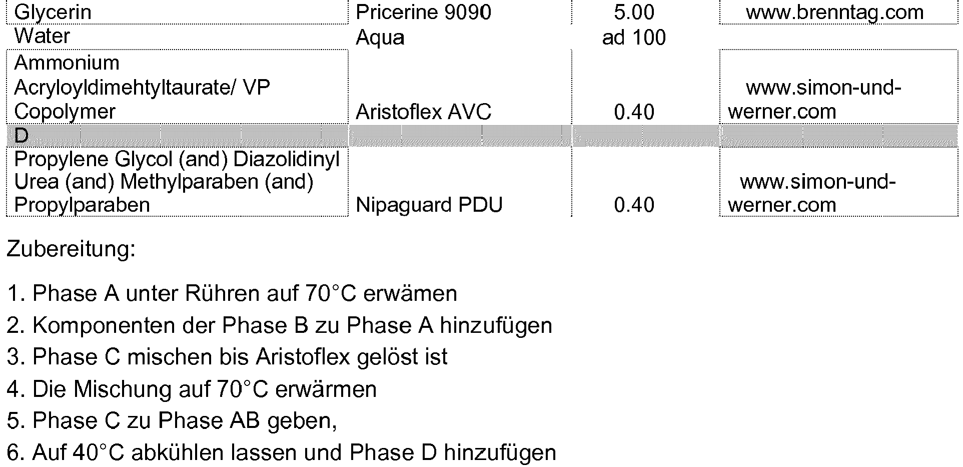

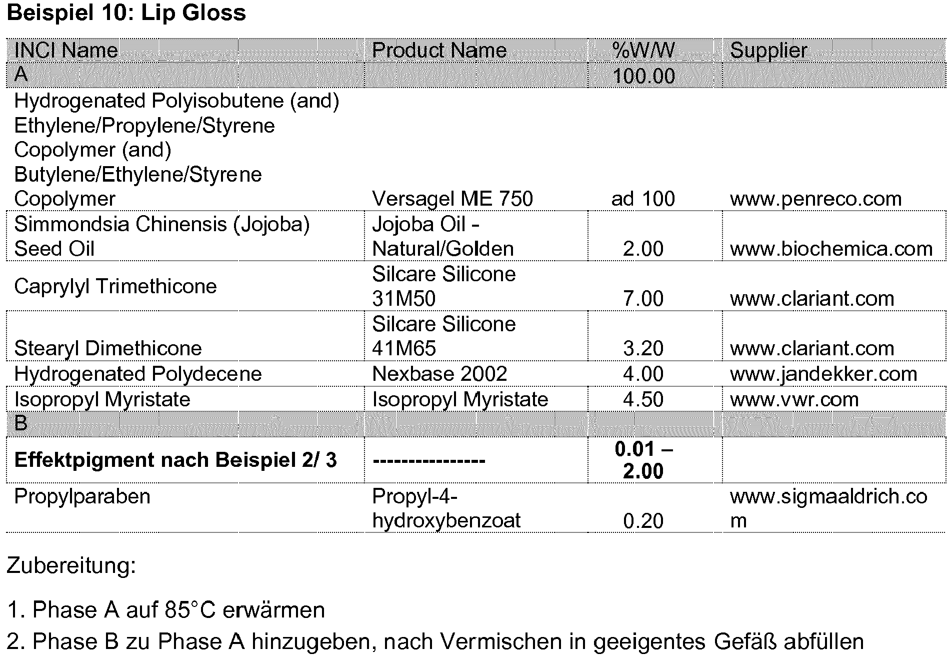

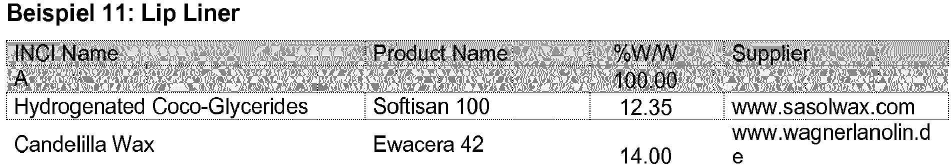

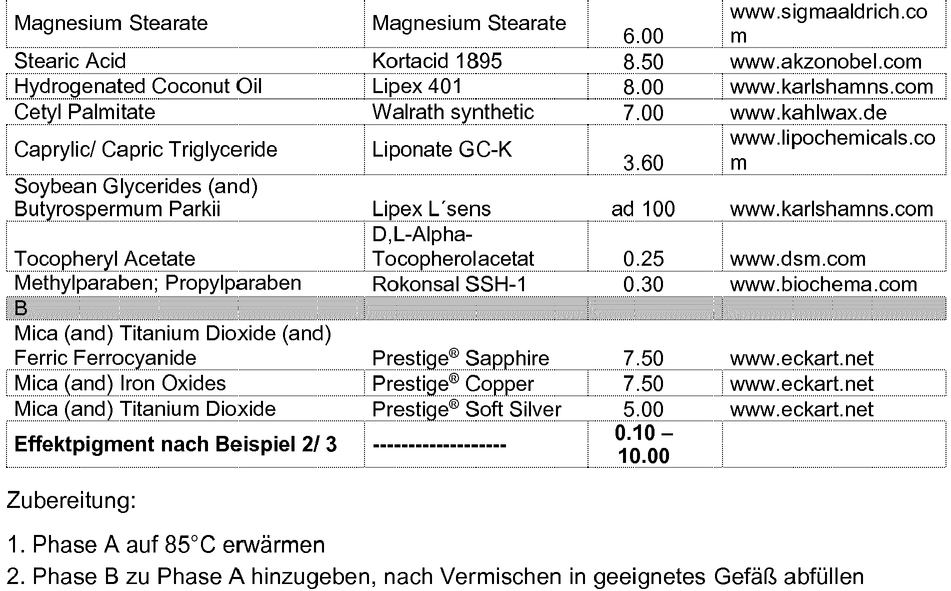

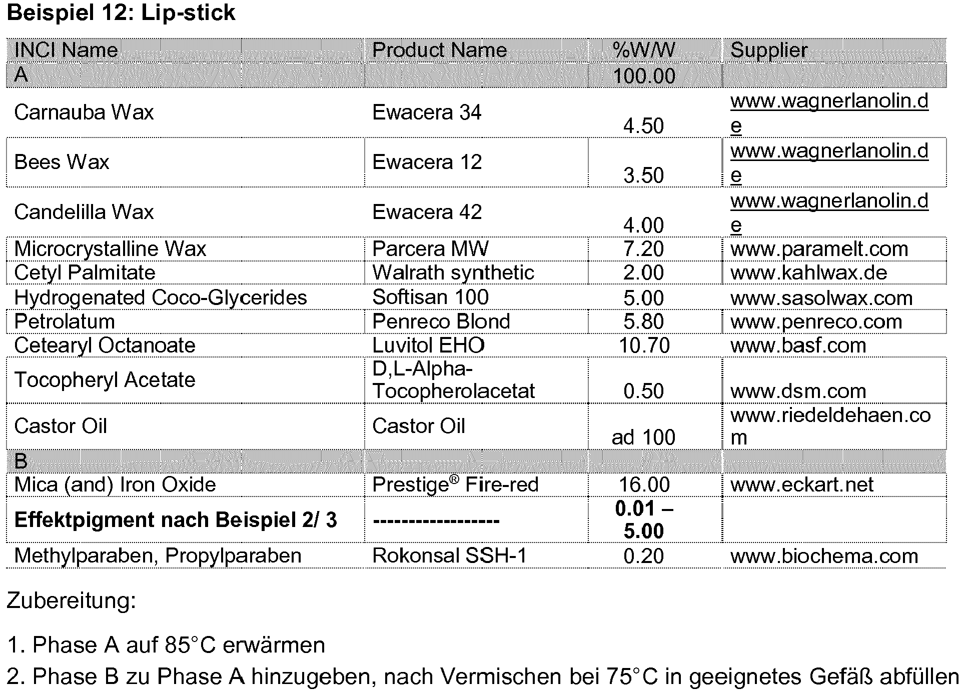

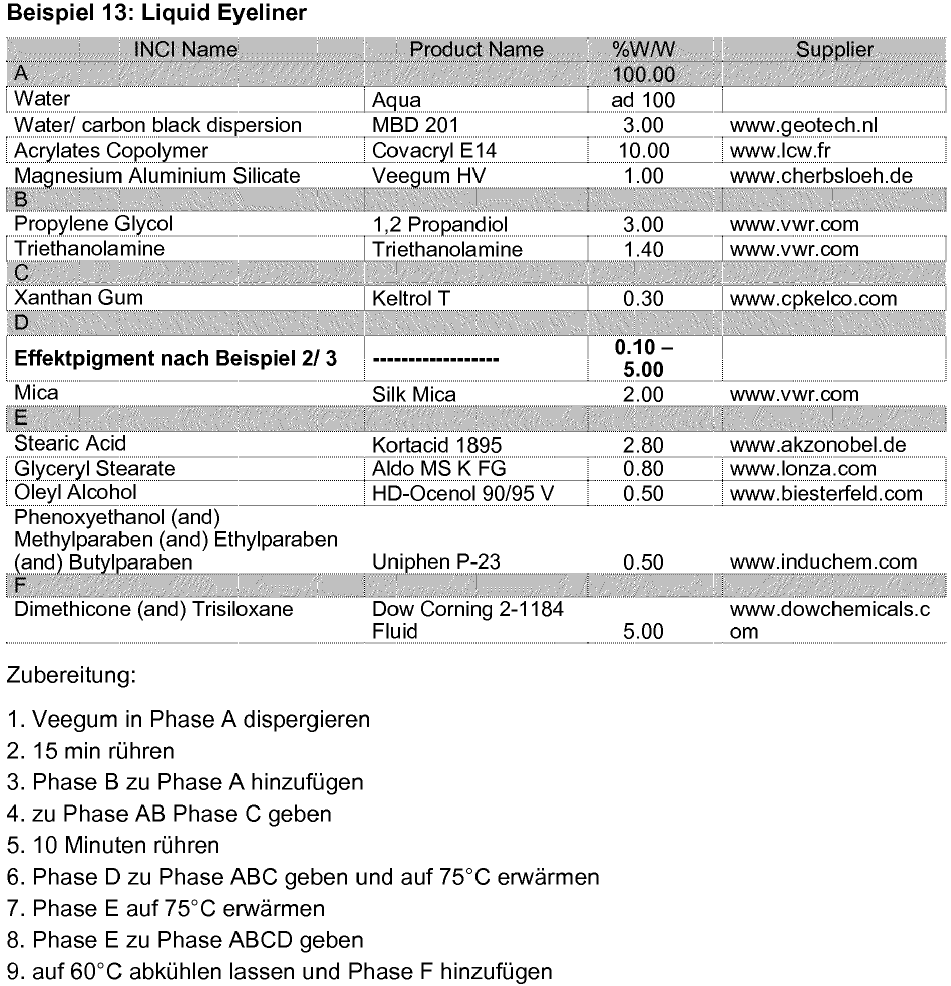

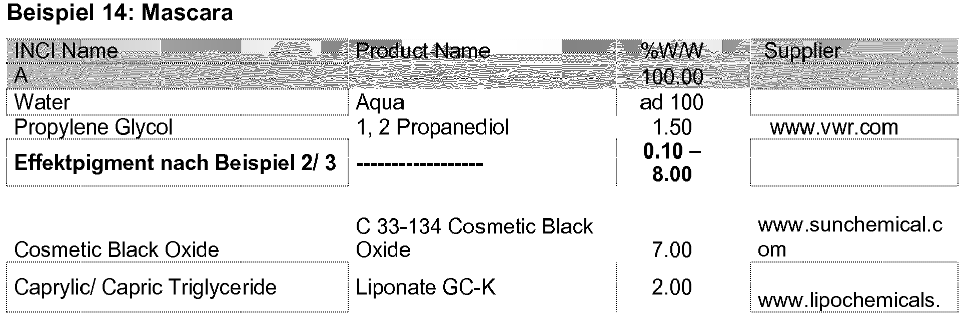

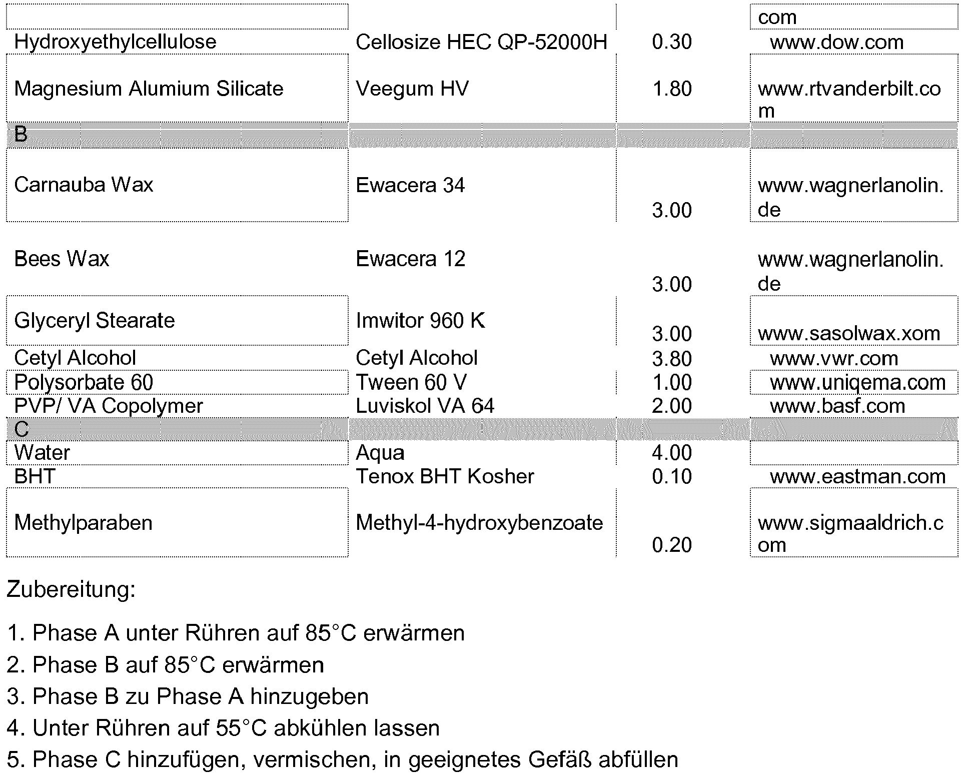

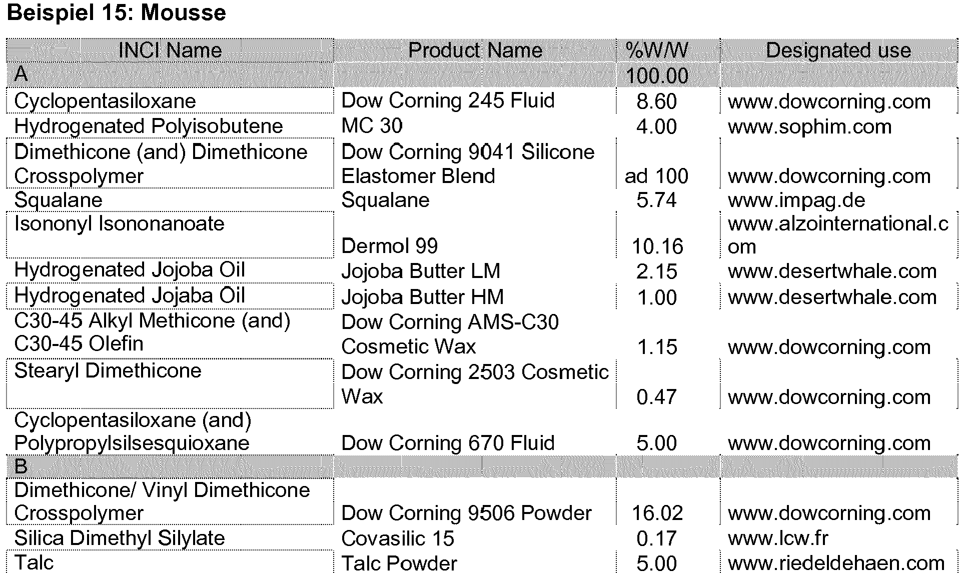

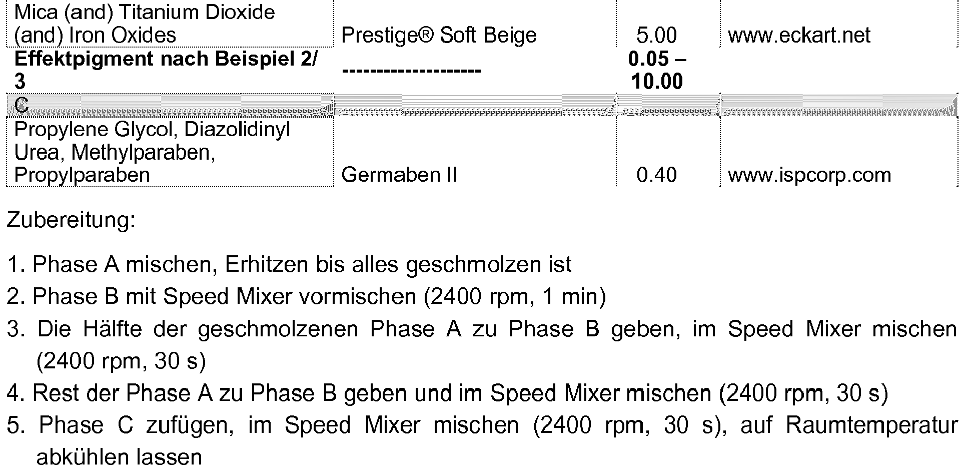

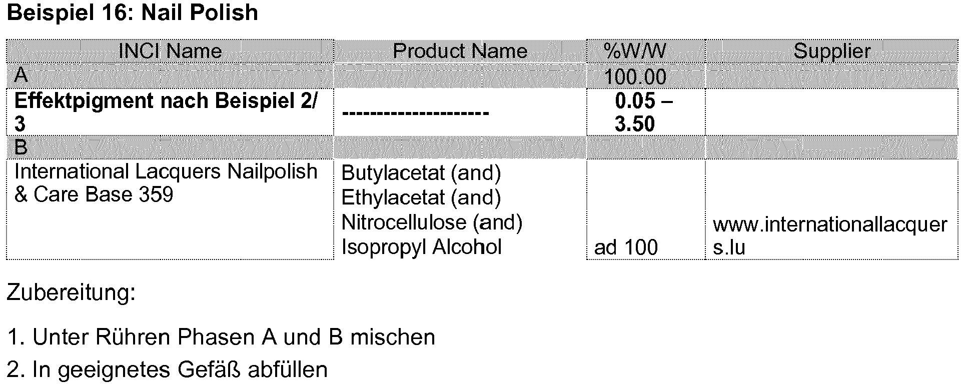

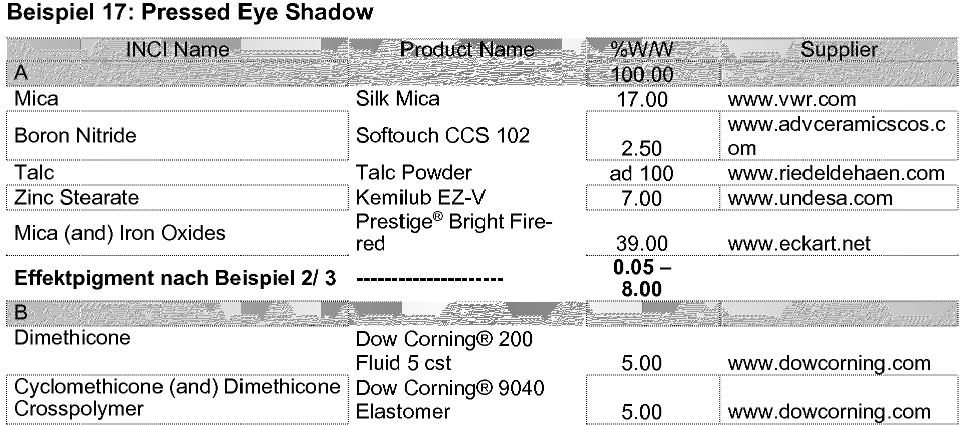

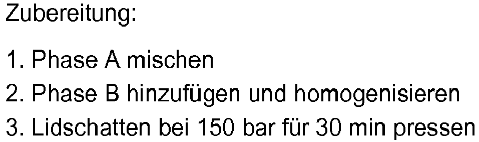

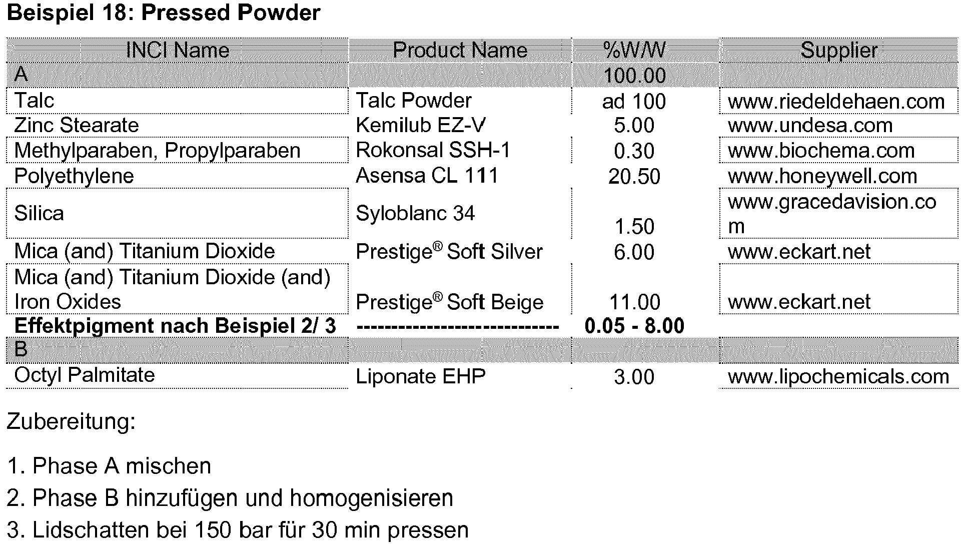

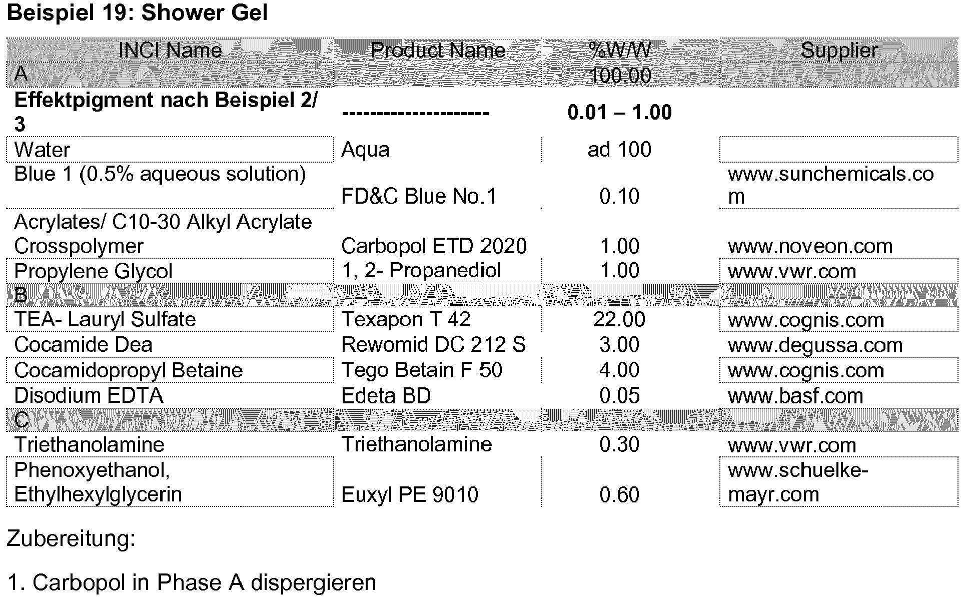

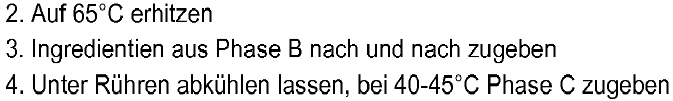

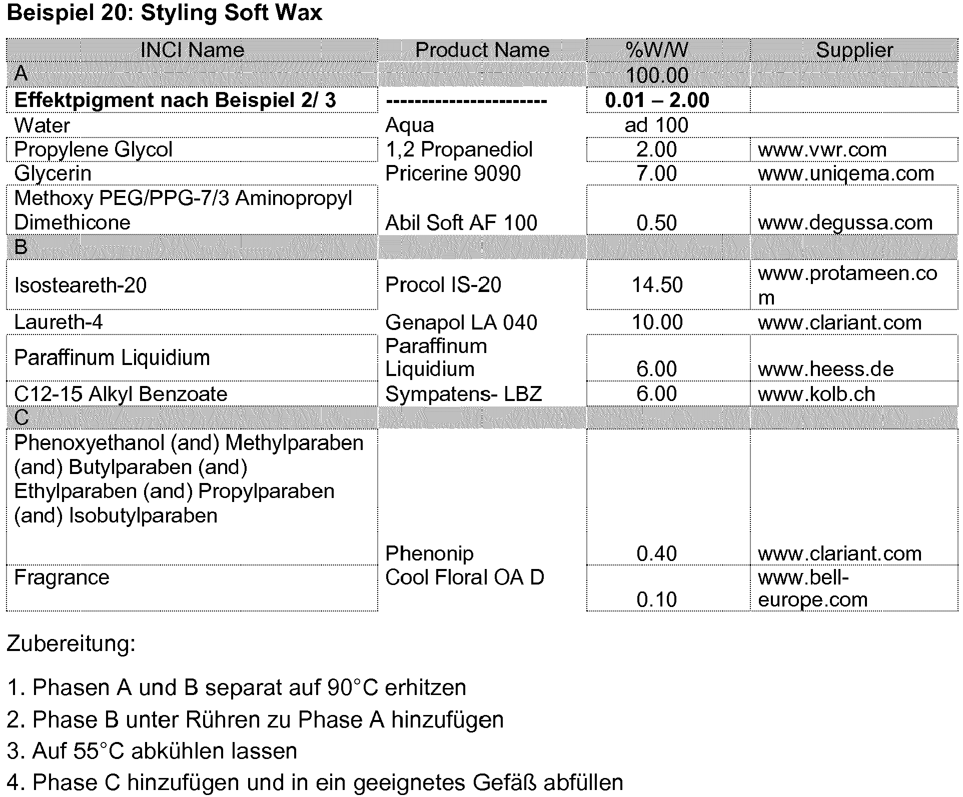

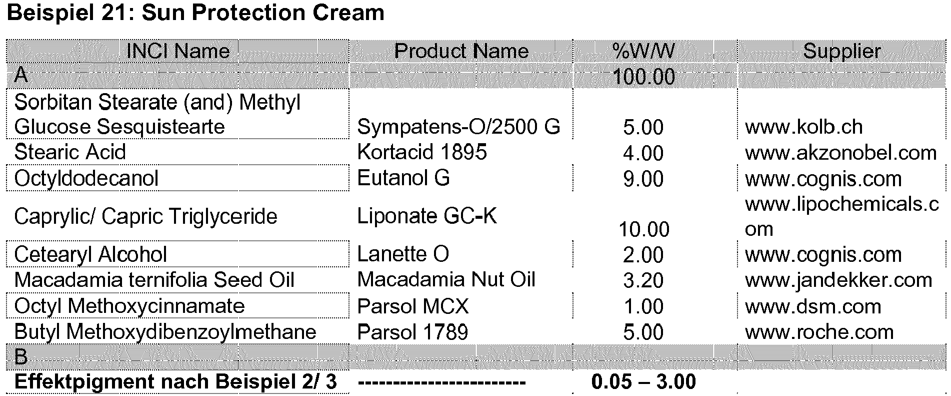

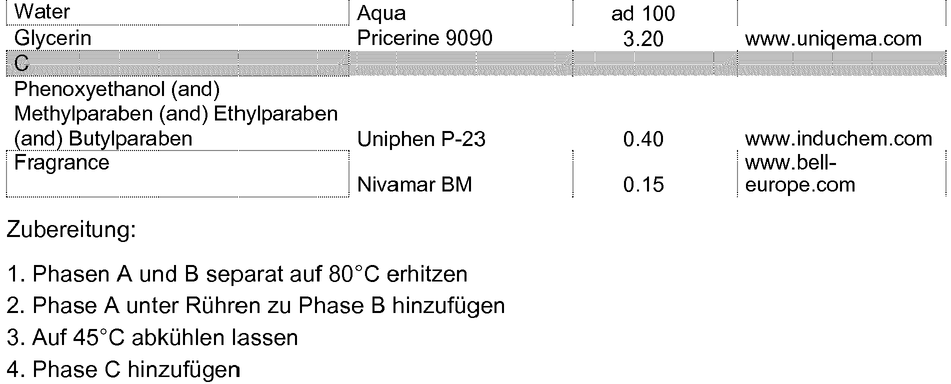

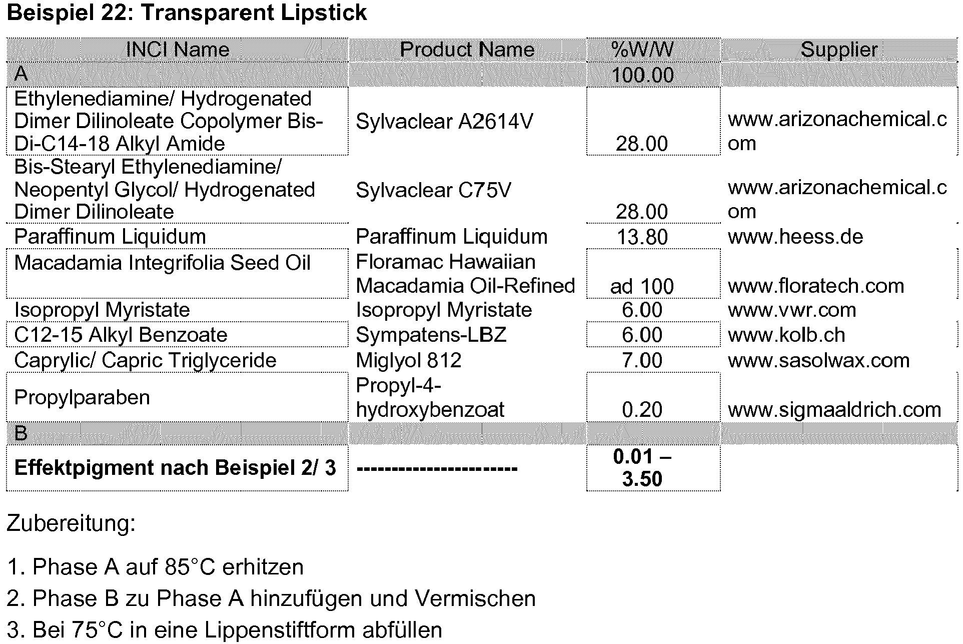

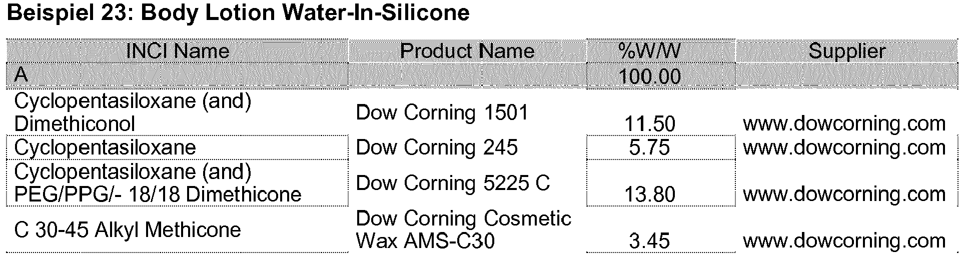

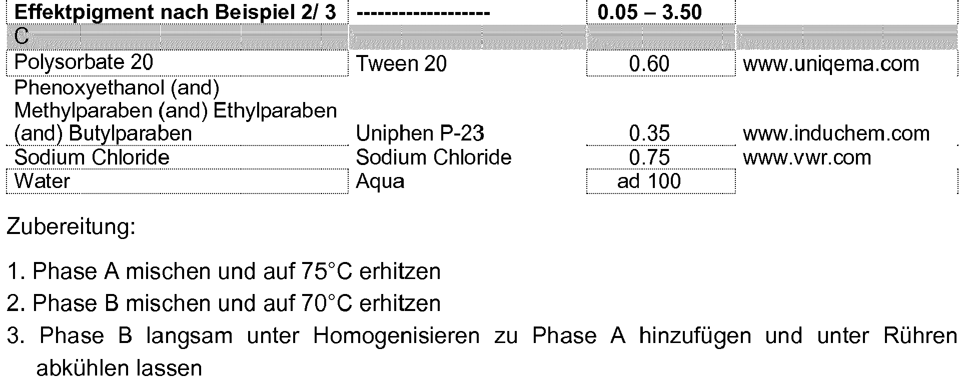

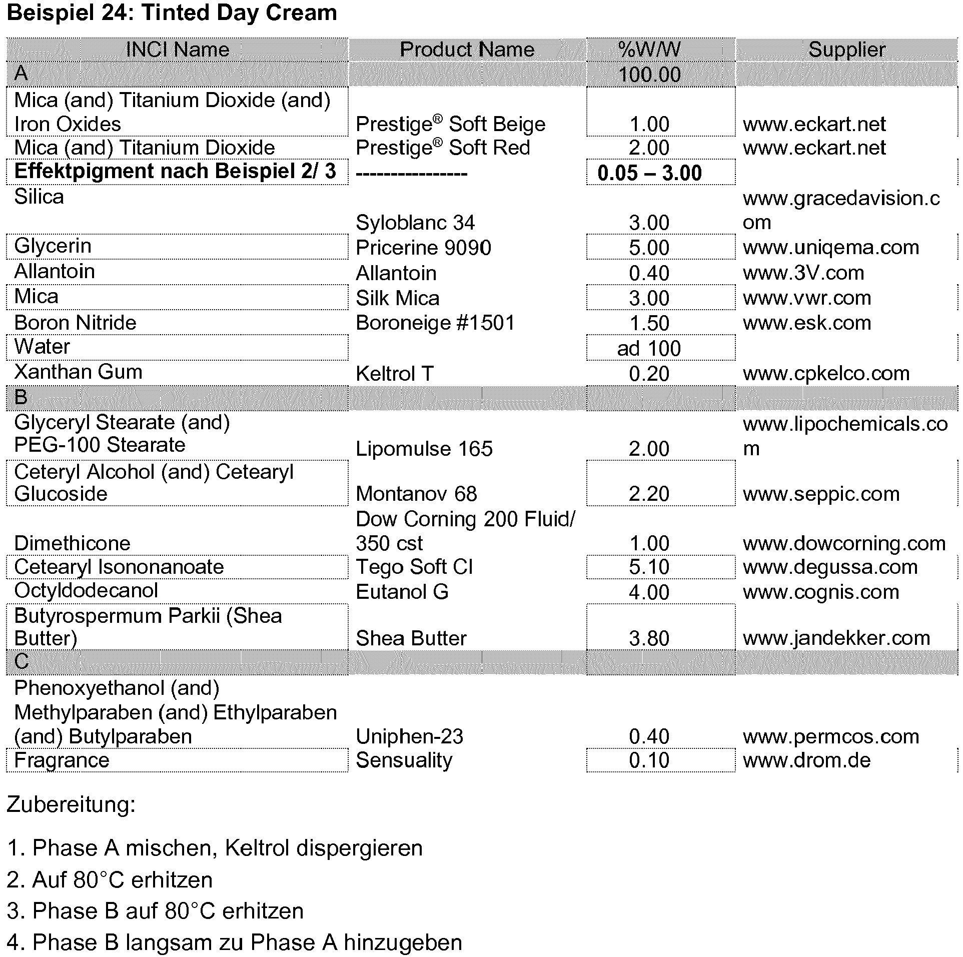

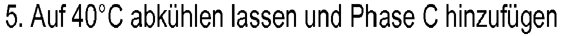

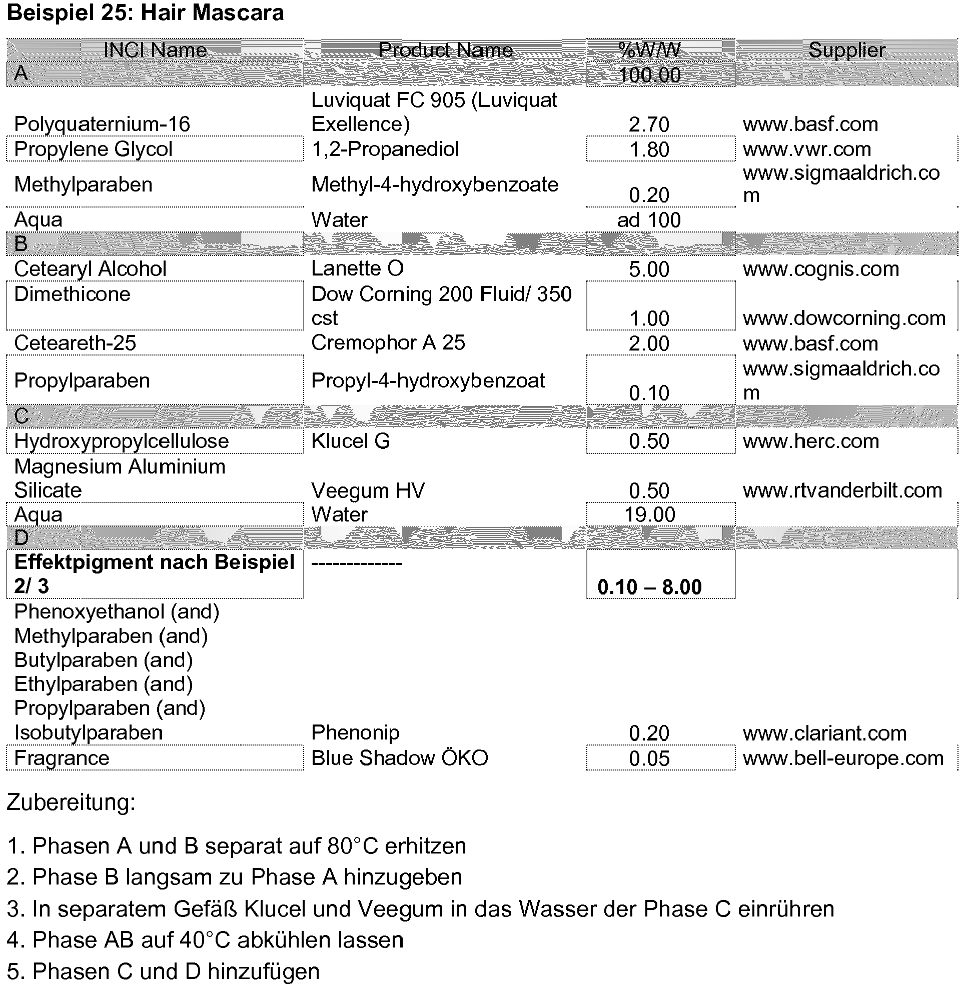

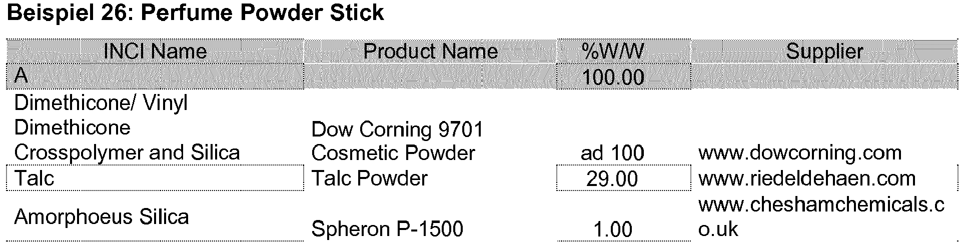

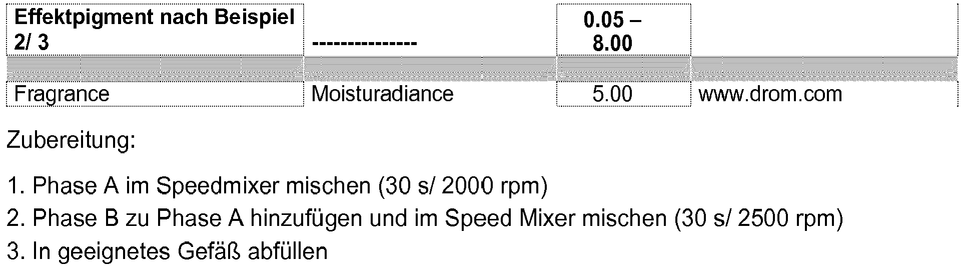

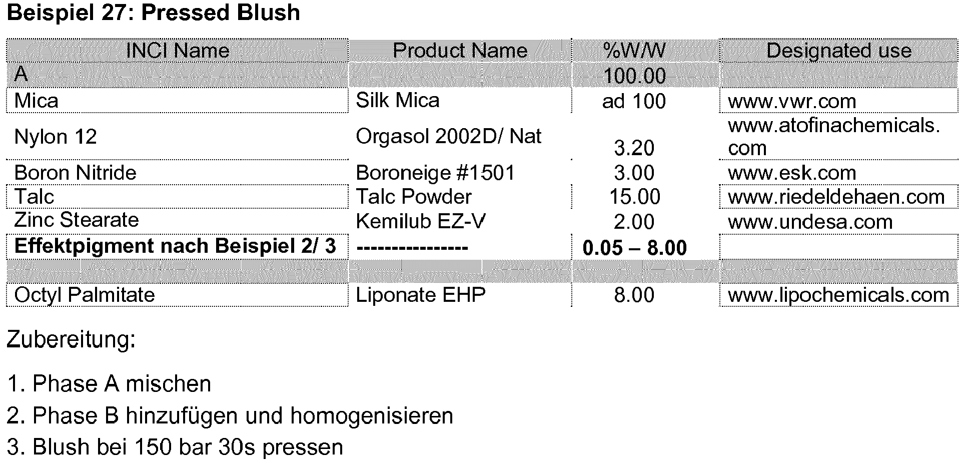

## Patentansprüche

1. Effektpigmente, umfassend künstliche plättchenförmige Substrate, die mindestens eine optisch wirksame Beschichtung aufweisen,
**dadurch gekennzeichnet,**
**dass** die Effektpigmente eine volumengemittelte Größensummendurchgangsverteilungskurve mit den Kennzahlen D10, D50 und D90 aufweisen, wobei diese Größensummendurchgangsverteilungskurve einen Span ΔD von 0,7 -1,4 aufweist und der Span ΔD gemäß Formel (I) berechnet ist: ΔD = (D90 - D10)/ D50, (I), wobei die mittlere Dicke der künstlichen plättchenförmigen Substrate 500 nm bis 2.000 nm und die Standardabweichung der Dicke der künstlichen Substrate 15 % bis 70 % beträgt.

2. Effektpigmente nach Anspruch 1,
**dadurch gekennzeichnet,**
**dass** die mittlere Dicke der künstlichen plättchenförmigen Substrate 750 nm bis 1.500 nm beträgt.

3. Effektpigmente nach einem der vorangehenden Ansprüche,
**dadurch gekennzeichnet,**
**dass** die Standardabweichung der Dicke der künstlichen Substrate 20 % bis 70 % beträgt.

4. Effektpigmente nach einem der vorangehenden Ansprüche,
**dadurch gekennzeichnet,**
**dass** die mindestens eine optisch wirksame Beschichtung der künstlichen Substrate Metalloxide, Metallhydroxide, Metalloxidhydrate, Metallsuboxide, Metalle, Metallfluoride, Metallnitride, Metalloxynitride oder Mischungen dieser Materialien umfasst.

5. Effektpigmente nach einem der vorangehenden Ansprüche,
**dadurch gekennzeichnet,**
**dass** die mindestens eine optisch wirksame Beschichtung der künstlichen Substrate einen Brechungsindex von mindestens n >1,9 aufweist.

6. Effektpigmente nach einem der vorangehenden Ansprüche,
**dadurch gekennzeichnet,**
**dass** die mindestens eine optisch wirksame Beschichtung einen Brechungsindex von n >1,9 aufweist und aus der Gruppe, die aus Metalloxid, Metallhydroxid, Metalloxidhydrat, Metallsuboxid und Mischungen davon besteht, ausgewählt wird.

7. Effektpigmente nach Anspruch 6,
**dadurch gekennzeichnet,**
**dass** die Effektpigmente in einem Rakelabzug eines Lackes, enthaltend 6 Gew.-% Effektpigmente in einem farblosen Nitrozelluloselack, bezogen auf das Gesamtgewicht des Lackes, mit 76 µm Naßfilmdicke nach dessen Trocknung ein 90% Quantil der Farbabstandsverteilung ΔC* 90 im a*-b*-Farbraum von 0,5 bis 8,0 aufweisen.

8. Effektpigmente nach einem der vorangehenden Ansprüche,
**dadurch gekennzeichnet,**
**dass** die Größensummendurchgangsverteilungskurve einen D50 -Wert in einem Bereich von 3 bis 350 µm aufweist.

9. Effektpigmente nach einem der vorangehenden Ansprüche,
**dadurch gekennzeichnet,**
**dass** die künstlichen Substrate im wesentlichen transparent sind und vorzugsweise aus der Gruppe, bestehend aus Glasplättchen, Plättchen aus synthetischem Glimmer, SiO2-Plättchen, Polymerplättchen, plättchenförmigem Bismuthoxychlorid, plättchenförmigen Aluminiumoxiden und Gemischen davon, ausgewählt werden.

10. Effektpigmente nach einem der vorangehenden Ansprüche,
**dadurch gekennzeichnet,**
**dass** die künstlichen Substrate Glasplättchen umfassen.

11. Verfahren zur Herstellung von Effektpigmenten nach einem der vorstehenden Ansprüche,
**dadurch gekennzeichnet,**
**dass** es folgende Schritte umfasst:
a) Größenklassieren der künstlichen Substrate
b) Beschichten der künstlichen Substrate mit wenigstens einer optisch wirksamen Beschichtung.

12. Verfahren zur Herstellung von Effektpigmenten nach Anspruch 11,
**dadurch gekennzeichnet,**
**dass** die Beschichtung der künstlichen Substrate in Schritt b) nach der Größenklassierung der künstlichen Substrate in Schritt a) erfolgt.

13. Verwendung der Effektpigmente nach einem der Ansprüche 1 bis 11 in Kosmetika, Kunststoffen und Beschichtungszusammensetzungen wie Farben, Druckfarben, Lacken, Pulverlacken, Elektrotauchlacken.

14. Verwendung der Effektpigmente nach Anspruch 13 in Kosmetika wie Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen sowie Nagellackkompositionen.
